# NEW EUROPEAN PATENT SPECIFICATION

(11) **EP 3 006 045 B3**
(45) Date of publication of this specification: **17.03.2021**
(45) Mention of the grant of the patent: 12.04.2017
(21) Application number: 14187885.0
(22) Date of filing: 07.10.2014
(51) Int. Cl.: A61K 47/02, A61K 38/28, A61K 47/12, A61K 9/48

(54) **Pharmaceutical formulations for the oral delivery of peptide or protein drugs**
Pharmazeutische Formulierungen zur oralen Verabreichung von Peptid- oder Proteinarzneimitteln
Formulations pharmaceutiques pour l'administration orale de médicaments peptidiques ou protéines

(43) Date of publication of application: 13.04.2016
(73) Proprietor: Cyprumed GmbH, 6416 Obsteig (AT)
(72) Inventor: Föger, Florian, 6416 Obsteig (AT)
(74) Representative: Vossius & Partner Patentanwälte Rechtsanwälte mbB

(56) References cited:
- WO-A1-2007/041481
- WO-A1-2007/062494
- US-A1- 2007 087 957
- LIND S.E. ET AL.: "Oxidative inactivation of plasmin and other serine proteases by copper and ascorbate", BLOOD, vol. 82, no. 5, 1 September 1993 (1993-09-01), pages 1522-1531, XP002734935,

## Description

The present invention relates to improved pharmaceutical formulations, uses and methods for the oral delivery of peptide or protein drugs with advantageously high bioavailability, safety and cost-effectiveness. In particular, the invention provides a peptide or protein drug having a molecular weight of equal to or less than about 50 kDa (as further defined in the claims) for use as a medicament, wherein said peptide or protein drug is to be administered orally in combination with a pharmaceutically acceptable copper salt/complex and/or a pharmaceutically acceptable zinc salt/complex, and with a pharmaceutically acceptable reducing agent. The invention also provides a pharmaceutical composition for use in therapy by oral administration, comprising: a peptide or protein drug having a molecular weight of equal to or less than about 50 kDa (as further defined in the claims); a pharmaceutically acceptable copper salt/complex and/or a pharmaceutically acceptable zinc salt/complex; and a pharmaceutically acceptable reducing agent.

A growing number of proteins and polypeptides have been made available as therapeutic agents. However, the full potential of these biological drugs has not been realized because they are limited to parenteral injection. Ideally, the oral route of administration would be preferred. Oral administration is the most common and popular method of administering drugs due to its simplicity and convenience for patients. However, the gastrointestinal tract degrades these macromolecules and prevents their absorption as intact entities. Enzymatic degradation throughout the gastrointestinal tract and poor permeability through the epithelial cells are the main reasons for their low oral bioavailability.

A number of different approaches have been proposed to improve the oral bioavailability of such therapeutic proteins and polypeptides, including the use of absorption enhancing technologies or the use of protease inhibitors such as soybean trypsin inhibitor, aprotinin, bowman birk inhibitor, bacitracin, camostat mesilate and amastatin (Renukuntla J et al., Int J Pharm. 2013, 447(1-2):75-93; US 2007/0087957 A1). However, due to safety concerns none of these protease inhibitors has succeeded as additive in commercial polypeptide drug delivery applications. The protease inhibitors described in known oral polypeptide drug delivery technologies and their toxicity and potential side effects are summarized in the following. Soybean trypsin inhibitor: Soy is widely accepted as one of "the big eight" allergens that causes immediate reactions such as coughing, sneezing, runny nose, hives, diarrhea, facial swelling, shortness of breath, swollen tongue, difficulty of swallowing, lowered blood pressure, excessive perspiration, fainting, anaphylactic shock and even death; the number of people suffering from soy allergies has been increasing steadily since the 1980s (Moroz LA et al., N Engl J Med. 1980, 302(20):1126-8; Foucard T et al., Allergy. 1999, 54(3):261-5; Ramesh S, Clin Rev Allergy Immunol. 2008, 34(2):217-30).

Bowman birk inhibitor: Another soybean derived protease inhibitor is the Bowman birk inhibitor. Bowman birk inhibitor is known to have oral bioavailability even without absorption enhancing additives and could therefore exert unwanted systemic protease inhibition after oral intake. Systemic inhibition of serine proteases such as plasmin could increase the risk of thrombosis. There are also reports about the formation of antibodies against bowman birk inhibitor (Wan XS et al., Nutr Cancer. 2002, 43(2):167-73).

Aprotinin: There have been several concerns about the safety of aprotinin. Anaphylaxis occurs at a rate of 1:200 in first-time use (Mahdy AM et al., Br J Anaesth. 2004, 93(6):842-58). A study performed in cardiac surgery patients reported in 2006 showed that there was a risk of acute renal failure, myocardial infarction and heart failure, as well as stroke and encephalopathy (Mangano DT et al., N Engl J Med. 2006, 354(4):353-65). Moreover, a study comparing aprotinin with aminocaproic acid found that mortality was increased by 64% (Schneeweiss S et al., N Engl J Med. 2008, 358(8):771-83).

The use of these protease inhibitors thus poses potential health risks and should preferably be avoided. Further disadvantages are high manufacturing costs, heterogeneity and regulatory hurdles. Furthermore, most protein based inhibitors have to be co-administrated excessively in large amounts because these compounds are susceptible to enzymatic degradation in the gut. Even large amounts of these inhibitors may not be adequate to reduce protease activity (Renukuntla J et al., Int J Pharm. 2013, 447(1-2):75-93).

It has also been proposed to use protease inhibitors such as bacitracin (having antibiotic activity), camostat mesilate (effective in the treatment of pancreatitis) or amastatin (having antibacterial activity) which, however, all have pharmacological effects on their own. Chronic administration of these protease inhibitors in oral polypeptide formulations would therefore not be acceptable (Renukuntla J et al., Int J Pharm. 2013, 447(1-2):75-93; US 2007/0087957 A1). Another disadvantage of protease inhibitors used so far in oral drug delivery systems is their limitation to inactivate just certain intestinal proteases. However, in order to efficiently deliver therapeutic polypeptide drugs in intact form via the oral route, more than just one or two of the intestinal serine proteases, such as trypsin, chymotrypsin, aminopeptidase, carboxypeptidase, elastase and dipeptidyl-4-peptidase, and also other enzymes such as insulin degrading enzyme need to be transiently inactivated. Otherwise, oral bioavailability will remain very low.

Thus, there is still an urgent need for simple, very safe, more efficient and less expensive means and methods to deliver therapeutic polypeptide drugs via the oral route.

It has further been described that aqueous solutions of copper in the presence of ascorbate reduce the activity of plasmin and other serine proteases in the blood (Lind SE et al., Blood. 1993, 82(5):1522-31). However, the use of copper and ascorbate in pharmaceutical compositions, let alone for the oral delivery of peptide or protein drugs, has never been proposed.

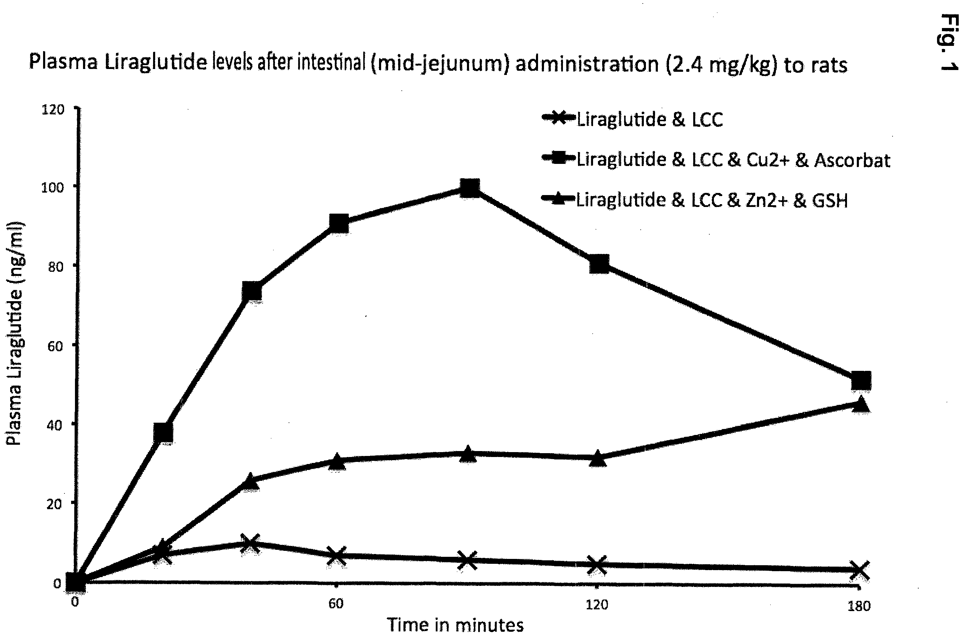

In the context of the present invention, it has been found that a combination of the trace element copper or zinc with a pharmaceutically acceptable reducing agent, optionally further in combination with a mucosal absorption enhancer that is soluble in the presence of the copper or zinc, results in a surprisingly high and advantageous oral bioavailability of different peptide or protein drugs, as also shown in the working examples (see, in particular, Example 6 and Figure 1). The required amounts of copper or zinc are well below the recommended daily intake levels of these trace elements and can therefore be regarded as safe. Moreover, copper or zinc in combination with a reducing agent exert an inhibitory effect on serine proteases in the gastrointestinal tract but do not show a systemic effect, which provides a further safety improvement as compared to the above-discussed protease inhibitors. Furthermore, copper or zinc as well as reducing agents such as ascorbate or reduced glutathione can be provided at considerably lower manufacturing costs than the above-discussed protease inhibitors that have previously been suggested for the oral delivery of peptide or protein drugs.

The present invention thus solves the problem of providing improved pharmaceutical formulations, uses and methods for the oral delivery of peptide or protein drugs, allowing the oral administration of a wide range of different peptide or protein drugs with advantageously high bioavailability, safety and cost-effectiveness.

Accordingly, in a first aspect, the present invention provides a peptide or protein drug having a molecular weight of equal to or less than about 50 kDa for use as a medicament, wherein said peptide or protein drug is to be administered orally in combination with: a pharmaceutically acceptable copper salt/complex and/or a pharmaceutically acceptable zinc salt/complex; and a pharmaceutically acceptable reducing agent.

As also defined in the claims, the "peptide or protein drug" to be used in the first aspect as well as any other aspect of the present invention is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

This definition applies whenever reference is made to a "peptide or protein drug" in the context of the present invention.

In accordance with this first aspect, the invention also relates to a peptide or protein drug having a molecular weight of equal to or less than about 50 kDa for use in therapy, wherein said peptide or protein drug is to be administered orally in combination with: a pharmaceutically acceptable copper salt/complex and/or a pharmaceutically acceptable zinc salt/complex; and a pharmaceutically acceptable reducing agent. The invention likewise provides a peptide or protein drug having a molecular weight of equal to or less than about 50 kDa for use in the treatment or prevention of a disease/disorder, wherein said peptide or protein drug is to be administered orally and in combination with: a pharmaceutically acceptable copper salt/complex and/or a pharmaceutically acceptable zinc salt/complex; and a pharmaceutically acceptable reducing agent. The invention further relates to a peptide or protein drug having a molecular weight of equal to or less than about 50 kDa for use as a medicament (or for use in therapy, or for use in the treatment or prevention of a disease/disorder), wherein said peptide or protein drug is to be administered orally in combination with a pharmaceutically acceptable copper salt/complex and a pharmaceutically acceptable reducing agent. Moreover, the present invention also provides a peptide or protein drug having a molecular weight of equal to or less than about 50 kDa for use as a medicament (or for use in therapy, or for use in the treatment or prevention of a disease/disorder), wherein said peptide or protein drug is to be administered orally in combination with a pharmaceutically acceptable zinc salt/complex and a pharmaceutically acceptable reducing agent. The invention furthermore relates to the use of a peptide or protein drug having a molecular weight of equal to or less than about 50 kDa in the preparation of a medicament which is to be administered orally in combination with: a pharmaceutically acceptable copper salt/complex and/or a pharmaceutically acceptable zinc salt/complex; and a pharmaceutically acceptable reducing agent. The invention likewise refers to the use of a peptide or protein drug having a molecular weight of equal to or less than about 50 kDa in the preparation of a medicament for the treatment or prevention of a disease/disorder, which is to be administered orally and in combination with: a pharmaceutically acceptable copper salt/complex and/or a pharmaceutically acceptable zinc salt/complex; and a pharmaceutically acceptable reducing agent.

In a second aspect, the present invention relates to a pharmaceutically acceptable copper salt/complex for use as a medicament (or for use in therapy, or for use in the treatment or prevention of a disease/disorder), wherein said copper salt/complex is to be administered orally in combination with: a pharmaceutically acceptable reducing agent; and a peptide or protein drug having a molecular weight of equal to or less than about 50 kDa.

In accordance with this second aspect, the invention also relates to the use of a pharmaceutically acceptable copper salt/complex in the preparation of a medicament which is to be administered orally in combination with: a pharmaceutically acceptable reducing agent; and a peptide or protein drug having a molecular weight of equal to or less than about 50 kDa. The invention further relates to the use of a pharmaceutically acceptable copper salt/complex in the preparation of a medicament for the treatment or prevention of a disease/disorder, which is to be administered orally in combination with: a pharmaceutically acceptable reducing agent; and a peptide or protein drug having a molecular weight of equal to or less than about 50 kDa.

In a third aspect, the invention provides a pharmaceutically acceptable zinc salt/complex for use as a medicament (or for use in therapy, or for use in the treatment or prevention of a disease/disorder), wherein said zinc salt/complex is to be administered orally in combination with: a pharmaceutically acceptable reducing agent; and a peptide or protein drug having a molecular weight of equal to or less than about 50 kDa.

In accordance with this third aspect, the invention further relates to the use of a pharmaceutically acceptable zinc salt/complex in the preparation of a medicament which is to be administered orally in combination with: a pharmaceutically acceptable reducing agent; and a peptide or protein drug having a molecular weight of equal to or less than about 50 kDa. The invention likewise relates to the use of a pharmaceutically acceptable zinc salt/complex in the preparation of a medicament for the treatment or prevention of a disease/disorder, which is to be administered orally in combination with: a pharmaceutically acceptable reducing agent; and a peptide or protein drug having a molecular weight of equal to or less than about 50 kDa.

In a fourth aspect, the present invention provides a pharmaceutically acceptable reducing agent for use as a medicament (or for use in therapy, or for use in the treatment or prevention of a disease/disorder), wherein said reducing agent is to be administered orally in combination with: a pharmaceutically acceptable copper salt/complex and/or a pharmaceutically acceptable zinc salt/complex; and a peptide or protein drug having a molecular weight of equal to or less than about 50 kDa.

In accordance with this fourth aspect, the invention also relates to a pharmaceutically acceptable reducing agent for use as a medicament (or for use in therapy, or for use in the treatment or prevention of a disease/disorder), wherein said reducing agent is to be administered orally in combination with a pharmaceutically acceptable copper salt/complex and a peptide or protein drug having a molecular weight of equal to or less than about 50 kDa. The invention likewise provides a pharmaceutically acceptable reducing agent for use as a medicament (or for use in therapy, or for use in the treatment or prevention of a disease/disorder), wherein said reducing agent is to be administered orally in combination with a pharmaceutically acceptable zinc salt/complex and a peptide or protein drug having a molecular weight of equal to or less than about 50 kDa. Moreover, the invention refers to the use of a pharmaceutically acceptable reducing agent in the preparation of a medicament which is to be administered orally in combination with: a pharmaceutically acceptable copper salt/complex and/or a pharmaceutically acceptable zinc salt/complex; and a peptide or protein drug having a molecular weight of equal to or less than about 50 kDa. The invention further relates to the use of a pharmaceutically acceptable reducing agent in the preparation of a medicament for the treatment or prevention of a disease/disorder, which is to be administered orally in combination with: a pharmaceutically acceptable copper salt/complex and/or a pharmaceutically acceptable zinc salt/complex; and a peptide or protein drug having a molecular weight of equal to or less than about 50 kDa.

In a fifth aspect, the present invention provides a pharmaceutical composition for use in therapy by oral administration, comprising: a peptide or protein drug having a molecular weight of equal to or less than about 50 kDa; a pharmaceutically acceptable copper salt/complex and/or a pharmaceutically acceptable zinc salt/complex; and a pharmaceutically acceptable reducing agent.

In accordance with this fifth aspect, the invention also relates to a pharmaceutical composition for use in therapy by oral administration, comprising: a peptide or protein drug having a molecular weight of equal to or less than about 50 kDa; a pharmaceutically acceptable copper salt/complex; and a pharmaceutically acceptable reducing agent. The invention likewise refers to a pharmaceutical composition for use in therapy by oral administration, comprising: a peptide or protein drug having a molecular weight of equal to or less than about 50 kDa; a pharmaceutically acceptable zinc salt/complex; and a pharmaceutically acceptable reducing agent. The pharmaceutical compositions of this fifth aspect are pharmaceutical compositions for oral administration.

In a sixth aspect, the invention provides a pharmaceutical dosage form for use in therapy by oral administration, comprising: a peptide or protein drug having a molecular weight of equal to or less than about 50 kDa; a pharmaceutically acceptable copper salt/complex and/or a pharmaceutically acceptable zinc salt/complex; and a pharmaceutically acceptable reducing agent; wherein the peptide or protein drug is physically separated from the pharmaceutically acceptable copper salt/complex and the pharmaceutically acceptable zinc salt/complex within the pharmaceutical dosage form. The pharmaceutical dosage form of this sixth aspect is a pharmaceutical dosage form for oral administration.

In a seventh aspect, the present invention provides a method of treating or preventing a disease/disorder, the method comprising orally administering, to a subject in need thereof, a peptide or protein drug having a molecular weight of equal to or less than about 50 kDa, a pharmaceutically acceptable copper salt/complex and/or a pharmaceutically acceptable zinc salt/complex, and a pharmaceutically acceptable reducing agent.

In accordance with this seventh aspect, the invention further relates to a method of orally delivering a peptide or protein drug having a molecular weight of equal to or less than about 50 kDa, the method comprising orally administering said peptide or protein drug in combination with a pharmaceutically acceptable copper salt/complex and/or a pharmaceutically acceptable zinc salt/complex and with a pharmaceutically acceptable reducing agent to a subject in need thereof. The invention also provides a method of facilitating the oral delivery of a peptide or protein drug having a molecular weight of equal to or less than about 50 kDa, the method comprising orally administering said peptide or protein drug in combination with a pharmaceutically acceptable copper salt/complex and/or a pharmaceutically acceptable zinc salt/complex and with a pharmaceutically acceptable reducing agent to a subject in need thereof. Furthermore, the invention relates to a method of administering a peptide or protein drug having a molecular weight of equal to or less than about 50 kDa, the method comprising orally administering said peptide or protein drug in combination with a pharmaceutically acceptable copper salt/complex and/or a pharmaceutically acceptable zinc salt/complex and with a pharmaceutically acceptable reducing agent to a subject in need thereof.

The following detailed description applies to all embodiments of the present invention, including all embodiments according to each one of the first, second, third, fourth, fifth, sixth and seventh aspect as described herein above.

The peptide or protein drug to be administered in accordance with the invention is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

Subject to the definition of the peptide or protein drug provided herein above (and in the claims), the peptide or protein drug has a molecular weight of equal to or less than about 50 kDa (such as, e.g., equal to or less than about 40 kDa, or equal to or less than about 30 kDa, or equal to or less than about 20 kDa, or equal to or less than about 10 kDa, or equal to or less than about 5 kDa, or equal to or less than about 2 kDa, or equal to or less than about 1 kDa, or equal to or less than about 500 Da). It is preferred that the peptide or protein drug has a maximum molecular weight of equal to or less than about 40 kDa, more preferably equal to or less than about 30 kDa, even more preferably equal to or less than about 20 kDa, and yet even more preferably equal to or less than about 10 kDa. It is furthermore preferred that the peptide or protein drug has a minimum molecular weight of equal to or greater than about 300 Da, more preferably equal to or greater than about 500 Da, even more preferably equal to or greater than about 800 Da, and yet even more preferably equal to or greater than about 1 kDa. Accordingly, it is particularly preferred that the peptide or protein drug has a molecular weight of about 300 Da to about 40 kDa, more preferably about 500 Da to about 30 kDa, even more preferably about 800 Da to about 20 kDa, and yet even more preferably about 1 kDa to about 10 kDa.

The molecular weight of the peptide or protein drug is indicated herein in dalton (Da), which is an alternative name for the unified atomic mass unit (u). A molecular weight of, e.g., 500 Da is thus equivalent to 500 g/mol. The term "kDa" (kilodalton) refers to 1000 Da.

The molecular weight of the peptide or protein drug can be determined using methods known in the art, such as, e.g., mass spectrometry (e.g., electrospray ionization mass spectrometry (ESI-MS) or matrix-assisted laser desorption/ionization mass spectrometry (MALDI-MS)), gel electrophoresis (e.g., polyacrylamide gel electrophoresis using sodium dodecyl sulfate (SDS-PAGE)), hydrodynamic methods (e.g., gel filtration chromatography or gradient sedimentation), or static light scattering (e.g., multi-angle light scattering (MALS)). It is preferred that the molecular weight of the peptide or protein drug is determined using mass spectrometry.

Subject to the definition of the peptide or protein drug provided herein above (and in the claims), the peptide or protein drug may be any peptide or protein that is suitable to be used as a medicament. For example, the peptide or protein drug may be a linear peptide or protein drug or a cyclic peptide or protein drug. It may also be a modified or derivatized peptide or protein drug, such as a PEGylated peptide or protein drug or a fatty acid acylated peptide or protein drug or a fatty diacid acylated peptide or protein drug. Moreover, the peptide or protein drug may be free of histidine residues and/or free of cysteine residues. It is generally preferred that the peptide or protein drug is water-soluble, particularly at neutral pH (i.e., at about pH 7). It is furthermore preferred that the peptide or protein drug has at least one serine protease cleavage site, i.e., that the peptide or protein drug comprises one or more amino acid residue(s) amenable or prone to cleavage by a serine protease (particularly an intestinal serine protease, such as trypsin, chymotrypsin, aminopeptidase, carboxypeptidase, elastase and/or dipeptidyl-4-peptidase). The term "peptide or protein drug" is used herein synonymously with "therapeutic peptide or protein" and "therapeutic peptide or protein drug".

In accordance with the present invention, the peptide or protein drug is: (i) a peptide or protein drug having a molecular weight of about 2 kDa to about 5 kDa, which is not glucagon; or (ii) a peptide or protein drug which is selected from GLP-1, a GLP-1 analog (e.g., an acylated GLP-1 analog or a diacylated GLP-1 analog), semaglutide, liraglutide, exenatide, lixizenatide, a dual agonist of the GLP-1 receptor and the glucagon receptor, amylin, an amylin analog, pramlintide, a somatostatin analog (e.g., octreotide, lanreotide, or pasireotide), goserelin (e.g., goserelin acetate), buserelin, leptin, a leptin analog (e.g., metreleptin), peptide YY (PYY), a PYY analog, glatiramer (e.g., glatiramer acetate), leuprolide, teriparatide, desmopressin, a glycopeptide antibiotic (e.g., a glycosylated cyclic or polycyclic nonribosomal peptide such as vancomycin, teicoplanin, telavancin, bleomycin, ramoplanin, or decaplanin), bortezomib, cosyntropin, chorionic gonadotropin, menotropin, sermorelin, luteinizing-hormone-releasing hormone (LHRH; also referred to as "gonadotropin-releasing hormone"), calcitonin (e.g., calcitonin-salmon), pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a (i.e., pegylated interferon alfa-2a), peginterferon alfa-2b (i.e., pegylated interferon alfa-2b), peginterferon beta-1a (i.e., pegylated interferon beta-1a), fibrinolysin, vasopressin, aldesleukin, darbepoetin alfa, filgrastim, interleukin-11, cyclosporine, urokinase, viomycin, thyrotropin-releasing hormone (TRH), leucine-enkephalin, methionine-enkephalin, substance P (CAS no. 33507-63-0), adrenocorticotropic hormone (ACTH), parathyroid hormone (PTH), and pharmaceutically acceptable salts thereof. If the subject/patient to be treated is a human and if the peptide or protein drug is an endogenous peptide or protein in human beings (i.e., occurs naturally in humans), it is furthermore preferred to use a human isoform of the corresponding peptide or protein (which may, e.g., be recombinantly expressed or chemically synthesized).

The peptide or protein drug to be used in accordance with the invention can also be a mixture of two or more different peptide or protein drugs, including the above-mentioned specific peptide or protein drugs.

The pharmaceutically acceptable "copper salt/complex" to be used in accordance with the present invention refers to a pharmaceutically acceptable salt of copper or a pharmaceutically acceptable complex (e.g., a chelate complex) of copper. Likewise, the pharmaceutically acceptable "zinc salt/complex" to be employed in accordance with the invention refers to a pharmaceutically acceptable salt of zinc or a pharmaceutically acceptable complex (e.g., a chelate complex) of zinc. While the expression "pharmaceutically acceptable" is omitted when referring to the copper salt/complex or the zinc salt/complex in the following, it will be understood that the corresponding salts or complexes to be used in accordance with the invention are pharmaceutically acceptable.

The copper salt/complex is preferably a copper(I) salt/complex or a copper(II) salt/complex. Exemplary copper(I) salts/complexes include copper(I) chloride (CuCI) and copper(I) acetate (CuCH₃CO₂). Exemplary copper(II) salts/complexes include copper sulfate (CuSO₄), copper carbonate (CuCO₃), copper(II) lysine complex, copper(II) citrate, and copper(II) gluconate. The copper salt/complex is more preferably a copper(II) salt/complex. The use of a copper(II) salt/complex is advantageous since it provides better aqueous solubility and better oxidation state stability than a copper(I) salt/complex. Even more preferably, the copper salt/complex to be used in accordance with the invention is a copper(II) salt/complex selected from copper sulfate (CuSO₄), copper carbonate (CuCO₃), copper(II) lysine complex (preferably Cu²⁺ L-lysine complex), copper(II) citrate, and copper(II) gluconate (preferably copper(II) D-gluconate).

The zinc salt/complex is preferably a zinc(II) salt/complex. Exemplary zinc(II) salts/complexes include zinc sulfate, zinc chloride, zinc acetate, zinc oxide, zinc ascorbate, zinc caprylate, zinc gluconate, zinc stearate, and zinc carbonate. Thus, the zinc salt/complex is more preferably selected from zinc sulfate, zinc chloride, zinc acetate, zinc oxide, zinc ascorbate, zinc caprylate, zinc gluconate, zinc stearate, and zinc carbonate.

While either a copper salt/complex or a zinc salt/complex (or both a copper salt/complex and a zinc salt/complex) can be employed in accordance with the present invention, the use of a copper salt/complex has been found to provide a greater improvement of oral bioavailability of the corresponding peptide or protein drug than the use of a zinc salt/complex, as also demonstrated in Example 6. The use of a copper salt/complex is thus preferred over the use of a zinc salt/complex. At the same time, the use of a zinc salt/complex is advantageous since zinc can safely be administered to humans at even higher doses than copper.

The pharmaceutically acceptable reducing agent to be used in accordance with the present invention is not particularly limited and may be any reducing agent that is acceptable for oral administration. It is preferred that the pharmaceutically acceptable reducing agent is selected from ascorbic acid (preferably an ascorbate, e.g., sodium ascorbate), reduced glutathione (GSH), cysteine, uric acid, a reducing sugar (preferably a reducing monosaccharide, such as, e.g., glucose, glyceraldehyde or galactose, or a reducing disaccharide, such as, e.g., lactose or maltose), mannitol, α-tocopherol, vitamin A, α-lipoic acid, dihydro-α-lipoic acid (DHLA), a thiol-bearing compound, a thiomer (also referred to as a "thiolated polymer"; may be synthesized, e.g., by immobilization of sulfhydryl bearing ligands on a polymeric backbone of well-established polymers such as, e.g., polyacrylic acid, carboxymethylcellulose or chitosan; exemplary thiomers include the thiomers that are described in Laffleur F et al., Future Med Chem. 2012, 4(17):2205-16 (doi: 10.4155/fmc.12.165) which is incorporated herein by reference), and pharmaceutically acceptable salts of any of the aforementioned agents. Mixtures of two or more reducing agents, including any of the above-described reducing agents (such as, e.g., ascorbate and reduced glutathione), can also be used.

In each one of the first to seventh aspects described herein, it is particularly preferred that the peptide or protein drug, the copper salt/complex and/or the zinc salt/complex, and the pharmaceutically acceptable reducing agent are orally administered in combination with an absorption enhancer (also referred to herein as a "gastrointestinal absorption enhancer"). The administration of an absorption enhancer improves or facilitates the mucosal absorption of the peptide or protein drug in the gastrointestinal tract and is advantageous particularly if the peptide or protein drug is a large molecule, e.g., a peptide or protein drug having a molecular weight of about 1 kDa or more.

The absorption enhancer is preferably selected to be compatible with the copper salt/complex and/or the zinc salt/complex that is/are used, which can readily be tested, e.g., as described in Example 1. In particular, it is preferred that the absorption enhancer is soluble in an aqueous medium at a pH of about 7 in the presence of the copper salt/complex and/or the zinc salt/complex that is/are used. The occurrence of precipitation or flocculation, as observed for certain combinations of a specific copper salt/complex and a specific absorption enhancer in an aqueous medium (see Example 1), is undesirable but does not rule out the use of a corresponding formulation in accordance with the invention.

The absorption enhancer may be, e.g., zwitter-ionic absorption enhancer or a non-ionic absorption enhancer. It is preferred that the absorption enhancer is selected from C₈₋₂₀ alkanoyl carnitine (preferably lauroyl carnitine, myristoyl carnitine or palmitoyl carnitine; e.g., lauroyl carnitine chloride, myristoyl carnitine chloride or palmitoyl carnitine chloride), salicylic acid (preferably a salicylate, e.g., sodium salicylate), a salicylic acid derivative (such as, e.g., 3-methoxysalicylic acid, 5-methoxysalicylic acid, or homovanillic acid, a C₈₋₂₀ alkanoic acid (preferably a C₈₋₂₀ alkanoate, more preferably a caprate, a caprylate, a myristate, a palmitate, or a stearate, such as, e.g., sodium caprate, sodium caprylate, sodium myristate, sodium palmitate, or sodium stearate), citric acid (preferably a citrate, e.g., sodium citrate), a fatty acid acylated amino acid (e.g., any of the fatty acid acylated amino acids described in US 2014/0056953 A1 which is incorporated herein by reference, including, without being limited thereto, sodium lauroyl alaninate, N-dodecanoyl-L-alanine, sodium lauroyl asparaginate, N-dodecanoyl-L-asparagine, sodium lauroyl aspartic acid, N-dodecanoyl-L-aspartic acid, sodium lauroyl cysteinate, N-dodecanoyl-L-cysteine, sodium lauroyl glutamic acid, N-dodecanoyl-L-glutamic acid, sodium lauroyl glutaminate, N-dodecanoyl-L-glutamine, sodium lauroyl glycinate, N-dodecanoyl-L-glycine, sodium lauroyl histidinate, N-dodecanoyl-L-histidine, sodium lauroyl isoleucinate, N-dodecanoyl-L-isoleucine, sodium lauroyl leucinate, N-dodecanoyl-L-leucine, sodium lauroyl methioninate, N-dodecanoyl-L-methionine, sodium lauroyl phenylalaninate, N-dodecanoyl-L-phenylalanine, sodium lauroyl prolinate, N-dodecanoyl-L-proline, sodium lauroyl serinate, N-dodecanoyl-L-serine, sodium lauroyl threoninate, N-dodecanoyl-L-threonine, sodium lauroyl tryptophanate, N-dodecanoyl-L-tryptophane, sodium lauroyl tyrosinate, N-dodecanoyl-L-tyrosine, sodium lauroyl valinate, N-dodecanoyl-L-valine, sodium lauroyl sarcosinate, N-dodecanoyl-L-sarcosine, sodium capric alaninate, N-decanoyl-L-alanine, sodium capric asparaginate, N-decanoyl-L-asparagine, sodium capric aspartic acid, N-decanoyl-L-aspartic acid, sodium capric cysteinate, N-decanoyl-L-cysteine, sodium capric glutamic acid, N-decanoyl-L-glutamic acid, sodium capric glutaminate, N-decanoyl-L-glutamine, sodium capric glycinate, N-decanoyl-L-glycine, sodium capric histidinate, N-decanoyl-L-histidine, sodium capric isoleucinate, N-decanoyl-L-isoleucine, sodium capric leucinate, N-decanoyl-L-leucine, sodium capric methioninate, N-decanoyl-L-methionine, sodium capric phenylalaninate, N-decanoyl-L-phenylalanine, sodium capric prolinate, N-decanoyl-L-proline, sodium capric serinate, N-decanoyl-L-serine, sodium capric threoninate, N-decanoyl-L-threonine, sodium capric tryptophanate, N-decanoyl-L-tryptophane, sodium capric tyrosinate, N-decanoyl-L-tyrosine, sodium capric valinate, N-decanoyl-L-valine, sodium capric sarcosinate, N-decanoyl-L-sarcosine, sodium oleoyl sarcosinate, sodium N-decylleucine, sodium stearoyl glutamate (e.g., Amisoft HS-11 P), sodium myristoyl glutamate (e.g., Amisoft MS-11), sodium lauroyl glutamate (e.g., Amisoft LS-11), sodium cocoyl glutamate (e.g., Amisoft CS-11), sodium cocoyl glycinate (e.g., Amilite GCS-11), sodium N-decyl leucine, sodium cocoyl glycine, sodium cocoyl glutamate, sodium lauroyl alaninate, N-dodecanoyl-L-alanine, sodium lauroyl asparaginate, N-dodecanoyl-L-asparagine, sodium lauroyl aspartic acid, N-dodecanoyl-L-aspartic acid, sodium lauroyl cysteinate, N-dodecanoyl-L-cysteine, sodium lauroyl glutamic acid, N-dodecanoyl-L-glutamic acid, sodium lauroyl glutaminate, N-dodecanoyl-L-glutamine, sodium lauroyl glycinate, N-dodecanoyl-L-glycine, sodium lauroyl histidinate, N-dodecanoyl-L-histidine, sodium lauroyl isoleucinate, N-dodecanoyl-L-isoleucine, sodium lauroyl leucinate, N-dodecanoyl-L-leucine, sodium lauroyl methinoninate, N-dodecanoyl-L-methionine, sodium lauroyl phenylalaninate, N-dodecanoyl-L-phenylalanine, sodium lauroyl prolinate, N-dodecanoyl-L-proline, sodium lauroyl serinate, N-dodecanoyl-L-serine, sodium lauroyl threoninate, N-dodecanoyl-L-threonine, sodium lauroyl tryptophanate, N-dodecanoyl-L-tryptophane, sodium lauroyl tyrosinate, N-dodecanoyl-L-tyrosine, sodium lauroyl valinate, N-dodecanoyl-L-valine, N-dodecanoyl-L-sarcosine, sodium capric alaninate, N-decanoyl-L-alanine, sodium capric asparaginate, N-decanoyl-L-asparagine, sodium capric aspartic acid, N-decanoyl-L-aspartic acid, Sodium capric cysteinate, N-decanoyl-L-cysteine, sodium capric glutamic acid, N-decanoyl-L-glutamic acid, sodium capric glutaminate, N-decanoyl-L-glutamine, sodium capric glycinate, N-decanoyl-L-glycine, sodium capric histidinate, N-decanoyl-L-histidine, sodium capric isoleucinate, N-decanoyl-L-isoleucine, sodium capric leucinate, N-decanoyl-L-leucine, sodium capric methioninate, N-decanoyl-L-methionine, sodium capric phenylalaninate, N-decanoyl-L-phenylalanine, sodium capric prolinate, N-decanoyl-L-proline, sodium capric serinate, N-decanoyl-L-serine, sodium capric threoninate, N-decanoyl-L-threonine, sodium capric tryptophanate, N-decanoyl-L-tryptophane, sodium capric tyrosinate, N-decanoyl-L-tyrosine, sodium capric valinate, N-decanoyl-L-valine, sodium capric sarcosinate, sodium oleoyl sarcosinate, and pharmaceutically acceptable salts of any of the aforementioned compounds; or, e.g., C₈₋₂₀ alkanoyl sarcosinate (e.g., a lauroyl sarcosinate, such as sodium lauroyl sarcosinate) or one of the 20 standard proteinogenic α-amino acids that is acylated with a C₈₋₂₀ alkanoic acid), an alkylsaccharide (e.g., a C₁₋₂₀ alkylsaccharide, such as, e.g., C₈₋₁₀ alkylpolysaccharide like Multitrope™ 1620-LQ-(MV); or, e.g., n-octyl-beta-D-glucopyranoside, or n-dodecyl-beta-D-maltoside), a cyclodextrine (e.g., α-cydodextrin, β-cydodextrin. γ-cyclodextrin, methyl-β-cyclodextrin, hydroxypropyl β-cyclodextrin, or sulfobutylether β-cyclodextrin), sodium N-[8-(2-hydroxybenzoyl)amino]caprylate (also referred to as "SNAC"), a thiomer (also referred to as a thiolated polymer; may be synthesized, e.g., by immobilization of sulfhydryl bearing ligands on a polymeric backbone of well-established polymers such as, e.g., polyacrylic acid, carboxymethylcellulose or chitosan; exemplary thiomers include the thiomers that are described in Laffleur F et al., Future Med Chem. 2012, 4(17):2205-16 (doi: 10.4155/fmc.12.165) which is incorporated herein by reference), a calcium chelating compound (e.g., ethylenediaminetetraacetic acid (EDTA), ethylene glycol tetraacetic acid (EGTA), sodium citrate, or polyacrylic acid), cremophor EL (also referred to as "Kolliphor EL"; CAS no. 61791-12-6), chitosan, N,N,N-trimethyl chitosan, benzalkonium chloride, bestatin, cetylpyridinium chloride, cetyltrimethylammonium bromide, a C₂₋₂₀ alkanol (e.g., ethanol, decanol, lauryl alcohol, myristyl alcohol, or palmityl alcohol), a C₈₋₂₀ alkenol (e.g., oleyl alcohol), a C₈₋₂₀ alkenoic acid (e.g., oleic acid), dextran sulfate, diethyleneglycol monoethyl ether (transcutol), 1-dodecylazacyclo-heptan-2-one (Azone®), ethyl caprylate, glyceryl monolaurate, lysophosphatidylcholine, menthol, a C₈₋₂₀ alkylamine, a C₈₋₂₀ alkenylamine (e.g., oleylamine), phosphatidylcholine, a poloxamer, polyethylene glycol monolaurate, polyoxyethylene, polypropylene glycol monolaurate, a polysorbate (e.g., polysorbate 80), a deoxycholate (e.g., sodium deoxycholate), sodium glycocholate, sodium glycodeoxycholate, sodium lauryl sulfate (SDS), a taurocholate (e.g., sodium taurocholate), a taurodeoxycholate (e.g., sodium taurodeoxycholate), sucrose laurate, a sulfoxide (e.g., a (C₁₋₁₀ alkyl)-(C₁₋₁₀ alkyl)-sulfoxide, such as, e.g., decyl methyl sulfoxide, or dimethyl sulfoxide), cyclopentadecalactone, 8-(N-2-hydroxy-5-chloro-benzoyl)-amino-caprylic acid (also referred to as "5-CNAC"), dodecyl-2-N,N-dimethylamino propionate (also referred to as "DDAIP"), D-α-tocopheryl polyethylene glycol-1000 succinate (also referred to as "TPGS"), and pharmaceutically acceptable salts of the aforementioned compounds. Mixtures of two or more absorption enhancers, including any of the above-described absorption enhancers, can also be used.

The (i) peptide or protein drug, (ii) the copper salt/complex and/or the zinc salt/complex, (iii) the pharmaceutically acceptable reducing agent, and (iv) the optionally used absorption enhancer may be administered simultaneously/concomitantly or sequentially. In the case of sequential administration, the copper salt/complex and/or the zinc salt/complex as well as the pharmaceutically acceptable reducing agent may be administered first, followed by the administration of the peptide or protein drug and the optionally used absorption enhancer (e.g., at least about 5 min after the first administration, preferably about 5 min to about 3 hours after the first administration, more preferably about 10 min to about 1 hour after the first administration). Also, the copper salt/complex and/or the zinc salt/complex as well as the pharmaceutically acceptable reducing agent and the optionally used absorption enhancer may be administered first, followed by the administration of the peptide or protein drug (e.g., at least about 5 min after the first administration, preferably about 5 min to about 3 hours after the first administration, more preferably about 10 min to about 1 hour after the first administration). In the case of simultaneous administration, the (i) peptide or protein drug, (ii) the copper salt/complex and/or the zinc salt/complex, (iii) the pharmaceutically acceptable reducing agent, and (iv) the optionally used absorption enhancer may be administered in the same pharmaceutical composition, or in two or more different/separate pharmaceutical compositions, or in two or more different/separate compartments of the same pharmaceutical dosage form, as also described further below.

The peptide or protein drug, the copper salt/complex and/or the zinc salt/complex, the pharmaceutically acceptable reducing agent, and the optionally used absorption enhancer can be administered, e.g., in the form of a pharmaceutical composition as described in the fifth aspect of the invention.

Certain reducing agents, such as ascorbate and reduced glutathione, are known to chemically degrade peptide or protein drugs (Stadtman ER, Am J Clin Nutr. 1991, 54(6 Suppl):1125S-1128S; Schmitz T et al., Amino Acids. 2006, 30(1):35-42). Moreover, ascorbate is also known to undergo autoxidation in the presence of copper (Buettner GR, Free Radic Res Commun. 1986, 1(6):349-53) and could therefore lose its activity during prolonged storage.

It is therefore preferred that the pharmaceutical composition according to the fifth aspect of the invention is a solid composition or a liquid substantially water-free composition. Such compositions are particularly advantageous as they provide an improved shelf-stability and thus enable prolonged storage periods. The liquid substantially water-free composition is preferably a liquid composition that contains less than about 5% (v/v) of water, more preferably less than about 3% (v/v) of water, even more preferably less than about 1% (v/v) of water, even more preferably less than about 0.5% (v/v) of water, yet even more preferably less than about 0.1% (v/v) of water, and is still more preferably free of water. Most preferably, the pharmaceutical composition of the fifth aspect is a solid composition (e.g., a tablet or a powder). It is furthermore preferred that the solid composition is substantially water-free, e.g., contains less than about 5% (w/w) of water, preferably less than about 3% (w/w) of water, more preferably less than about 1% (w/w) of water, even more preferably less than about 0.5% (w/w) of water, yet even more preferably less than about 0.1% (w/w) of water, and is still more preferably free of water.

It is also possible, although not preferred, that the pharmaceutical composition according to the fifth aspect of the invention is an aqueous liquid composition (e.g., an aqueous solution). In this case, the composition should preferably be prepared shortly before administration to the subject/patient, and prolonged storage periods should be avoided.

Furthermore, it is particularly preferred that the pharmaceutical composition according to the fifth aspect is a pharmaceutical dosage form in which the peptide or protein drug is physically separated from the pharmaceutically acceptable copper salt/complex (if present) and the pharmaceutically acceptable zinc salt/complex (if present), as described in the sixth aspect of the invention.

The pharmaceutical dosage form according to the sixth aspect of the invention preferably comprises at least two separate compartments which are physically separated from one another (e.g., through a physical separation layer). Accordingly, it is preferred that the pharmaceutical dosage form comprises a physical separation layer between (i) the peptide or protein drug and (ii) the copper salt/complex (if present) and the zinc salt/complex (if present). The peptide or protein drug is present only in a first compartment, and the copper salt/complex and/or the zinc salt/complex is/are present only in a second compartment of the pharmaceutical dosage form. The pharmaceutically acceptable reducing agent may be present either in the first compartment, or in the second compartment, or in both the first and the second compartment, or in a third compartment of the pharmaceutical dosage form. In one preferred embodiment according to the sixth aspect, the invention thus provides a pharmaceutical dosage form (e.g., a double capsule) for use in therapy by oral administration, comprising: a peptide or protein drug having a molecular weight of equal to or less than about 50 kDa, which is present in a first compartment of the pharmaceutical dosage form; a pharmaceutically acceptable copper salt/complex and/or a pharmaceutically acceptable zinc salt/complex, which is/are present in a second compartment of the pharmaceutical dosage form; and a pharmaceutically acceptable reducing agent, which is present in the first compartment and/or the second compartment of the pharmaceutical dosage form. In a further preferred embodiment of the sixth aspect, the invention provides a pharmaceutical dosage form (e.g., a multi-particulate dosage form) for use in therapy by oral administration, comprising: a peptide or protein drug having a molecular weight of equal to or less than about 50 kDa, which is present in a first compartment of the pharmaceutical dosage form; a pharmaceutically acceptable reducing agent, which is present in a second compartment of the pharmaceutical dosage form; and a pharmaceutically acceptable copper salt/complex and/or a pharmaceutically acceptable zinc salt/complex, which is/are present in a third compartment of the pharmaceutical dosage form. It is particularly preferred that the pharmaceutical dosage form of the sixth aspect is a capsule inside a capsule (also referred to as a double capsule) or a multi-particulate dosage form. In the case of a double capsule, it is preferred that the bigger outer capsule (the content of which will be released first) contains the copper salt/complex and/or the zinc salt/complex as well as the pharmaceutically acceptable reducing agent, and that the smaller inner capsule (the content of which will be released later) contains the peptide or protein drug. The dosage form may also be a release-modified dosage form, such as a dosage form (e.g., a capsule, multiparticulate or tablet) having an enteric coating or a dosage form (e.g., a capsule, multiparticulate or tablet) coated with Eudragit L30D55 or with Eudragit FS30D or an acid resistant capsule such as HPMCP capsules (commercially known as AR Caps®).

The pharmaceutical composition according to the fifth aspect and also the pharmaceutical dosage form according to the sixth aspect of the invention preferably comprise the copper salt/complex in an amount of about 0.1 mg to about 5 mg per dosage unit, and/or the zinc salt/complex in an amount of about 0.1 mg to about 50 mg per dosage unit. They further comprise the pharmaceutically acceptable reducing agent in an amount of preferably about 1 mg to about 1000 mg per dosage unit, more preferably about 50 mg to about 500 mg per dosage unit. Moreover, if they comprise an absorption enhancer, the absorption enhancer is preferably included in an amount of about 10 mg to about 1000 mg per dosage unit, more preferably about 50 mg to about 500 mg per dosage unit.

It is furthermore preferred that the constitution of the pharmaceutical composition is such that, if the composition were added to ten milliliters of 5% HCI solution, it would neutralize the acid and generate a pH of higher than about 6. In addition, it is also preferred that the constitution of the pharmaceutical composition is such that, if the composition were added to ten milliliters of aqueous solution, it would generate a pH of higher than about 6 and lower than about pH 9.

The pharmaceutically acceptable salts referred to herein may be formed, e.g., by protonation of an atom carrying an electron lone pair which is susceptible to protonation, such as an amino group, with an inorganic or organic acid, or as a salt of a carboxylic acid group with a physiologically acceptable cation as they are well-known in the art. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, glycolate, nicotinate, benzoate, salicylate, ascorbate, or pamoate (embonate) salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; and acidic amino acid salts such as aspartate or glutamate salts. It is to be understood that the term "pharmaceutically acceptable salt" also embraces pharmaceutically acceptable salts of the corresponding compound in any solvated form.

The peptide or protein drug, the copper salt/complex and/or the zinc salt/complex, the pharmaceutically acceptable reducing agent, and the optionally used absorption enhancer (which are collectively referred to as the "compounds to be administered" in the following) may each be administered as compounds *per se* or may be formulated as medicaments, e.g., in the form of a pharmaceutical composition according to the fifth aspect and/or a pharmaceutical dosage form according to the sixth aspect of the invention. The medicaments/pharmaceutical compositions, including also the pharmaceutical composition according to the fifth aspect and the pharmaceutical dosage form according to the sixth aspect, may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers. In particular, they may comprise one or more additives selected from vitamin E, histidine, microcrystalline cellulose (MCC), mannitol, starch, sorbitol and/or lactose. The pharmaceutical compositions can be formulated by techniques known to the person skilled in the art, such as the techniques published in Remington's Pharmaceutical Sciences, 20th Edition.

As noted above, the pharmaceutical compositions may comprise one or more solubility enhancers, such as, e.g., poly(ethylene glycol), including poly(ethylene glycol) having a molecular weight in the range of about 200 to about 5,000 Da, ethylene glycol, propylene glycol, non-ionic surfactants, tyloxapol, polysorbate 80, macrogol-15-hydroxystearate, phospholipids, lecithin, dimyristoyl phosphatidylcholine, dipalmitoyl phosphatidylcholine, distearoyl phosphatidylcholine, cyclodextrins, α-cydodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, carboxyalkyl thioethers, hydroxypropyl methylcellulose, hydroxypropylcellulose, polyvinylpyrrolidone, vinyl acetate copolymers, vinyl pyrrolidone, sodium lauryl sulfate, dioctyl sodium sulfosuccinate, or any combination thereof.

The pharmaceutical compositions are formulated as dosage forms for oral administration, particularly peroral administration. Accordingly, the compounds to be administered or the above described pharmaceutical compositions, including also the pharmaceutical composition according to the fifth aspect and the pharmaceutical dosage form according to the sixth aspect, are administered to a subject/patient orally, particularly perorally. It is thus in accordance with the invention that the peptide or protein drug, the copper salt/complex and/or the zinc salt/complex, the pharmaceutically acceptable reducing agent, and the optionally used absorption enhancer are all to be administered orally.

Dosage forms for oral administration include, e.g., tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Typically, a physician will determine the actual dosage which will be most suitable for an individual subject. The specific dose level and frequency of dosage for any particular individual subject may be varied and will depend upon a variety of factors including the activity of the specific peptide or protein drug employed, the metabolic stability and length of action of that compound, the age, body weight, general health, sex, diet, mode and time of administration, rate of excretion, drug combination, the severity of the particular condition, and the individual subject undergoing therapy. The precise dose will ultimately be at the discretion of the attendant physician or veterinarian.

The subject or patient to be treated, such as the subject in need of treatment or prevention, may be an animal (e.g., a non-human animal), a vertebrate animal, a mammal, a rodent (e.g., a guinea pig, a hamster, a rat, a mouse), a murine (e.g., a mouse), a canine (e.g., a dog), a feline (e.g., a cat), a porcine (e.g., a pig), an equine (e.g., a horse), a primate, a simian (e.g., a monkey or ape), a monkey (e.g., a marmoset, a baboon), an ape (e.g., a gorilla, chimpanzee, orang-utan, gibbon), or a human. In the context of this invention, it is also envisaged that animals are to be treated which are economically or agronomically important. Non-limiting examples of agronomically important animals are sheep, cattle and pigs, while, for example, cats and dogs may be considered as economically important animals. Preferably, the subject/patient is a mammal; more preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orang-utan, a gibbon, a sheep, cattle, or a pig); most preferably, the subject/patient is a human.

The term "treatment" of a disorder or disease as used herein is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia*, comprise curative treatment (preferably leading to a complete response and eventually to healing of the disorder or disease) and palliative treatment (including symptomatic relief).

The term "prevention" of a disorder or disease as used herein is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of a peptide or protein drug according to the invention before any clinical and/or pathological symptoms are diagnosed or determined or can be diagnosed or determined by the attending physician.

The terms "peptide" and "protein", as in the expression "peptide or protein drug", are used herein interchangeably and refer to a polymer of two or more amino acids linked via amide bonds that are formed between an amino group of one amino acid and a carboxyl group of another amino acid. The amino acids comprised in the peptide or protein, which are also referred to as amino acid residues, may be selected from the 20 standard proteinogenic α-amino acids (i.e., Ala, Arg, Asn, Asp, Cys, Glu, Gln, Gly, His, Ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, or 4-hydroxyproline) as well as β-amino acids (e.g., β-alanine), γ-amino acids and δ-amino acids. Preferably, the amino acid residues comprised in the peptide or protein are selected from α-amino acids, more preferably from the 20 standard proteinogenic α-amino acids (which can be present as the L-isomer or the D-isomer, and are preferably all present as the L-isomer). The peptide or protein may be unmodified or may be modified, e.g., at its N-terminus, at its C-terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PG & Greene TW, Greene's protective groups in organic synthesis, John Wiley & Sons, 2006. Such modifications may also include the covalent attachment of one or more polyethylene glycol (PEG) chains (forming a PEGylated peptide or protein), the glycosylation and/or the acylation with one or more fatty acids (e.g., one or more C₈₋₃₀ alkanoic or alkenoic acids; forming a fatty acid acylated peptide or protein). The amino acid residues comprised in the peptide or protein may, e.g., be present as a linear molecular chain (forming a linear peptide or protein) or may form one or more rings (corresponding to a cyclic peptide or protein). The peptide or protein may also form oligomers consisting of two or more identical or different molecules.

As used herein, the term "complex" refers to a chelate complex (in which coordinate bonds are formed between a single central atom/ion and a polydentate ligand) or a coordination complex composed of monodentate ligands coordinating a single central atom/ion.

As used herein, the term "reducing sugar" refers to a sugar that has an open-chain form with an aldehyde group or a free hemiacetal group and can thus act as a reducing agent. A reducing sugar may be, e.g., a reducing monosaccharide (e.g., glucose, glyceraldehyde or galactose) or a reducing disaccharide (e.g., lactose or maltose).

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, if a component of a composition is indicated to be "optional", the invention specifically relates to both possibilities, i.e., that the corresponding component is present (contained in the composition) or that the corresponding component is absent from the composition.

As used herein, the term "about" refers to ±10% of the indicated numerical value, preferably to ±5% of the indicated numerical value, and in particular to the exact numerical value indicated. For example, the expression "about 100" refers to the range of 90 to 110, in particular the range of 95 to 105, and preferably refers to the specific value of 100. If the term "about" is used in connection with the endpoints of a range, it refers to the range from the lower endpoint -10% of its indicated numerical value to the upper endpoint +10% of its indicated numerical value, in particular to the range from of the lower endpoint -5% to the upper endpoint +5%, and preferably to the range defined by the exact numerical values of the lower endpoint and the upper endpoint. Thus, the expression "about 10 to about 20" refers to the range of 9 to 22, in particular 9.5 to 21, and preferably 10 to 20. If the term "about" is used in connection with the endpoint of an open-ended range, it refers to the corresponding range starting from the lower endpoint -10% or from the upper endpoint +10%, in particular to the range starting from the lower endpoint -5% or from the upper endpoint +5%, and preferably to the open-ended range defined by the exact numerical value of the corresponding endpoint. For example, the expression "at least about 10%" refers to at least 9%, particularly at least 9.5%, and preferably at least 10%.

Furthermore, it is to be understood that the present invention specifically relates to each and every combination of features and embodiments described herein, including any combination of general and/or preferred features/embodiments. In particular, the invention specifically relates to all combinations of preferred features described herein.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The invention is also described by the following illustrative figures. The appended figures show:
**Figure 1****:** Plasma liraglutide levels after intestinal (mid-jejunum) administration of different liraglutide formulations to rats (see Example 6).
**Figure 2****:** Blood glucose levels after intestinal (mid-jejunum) administration of different formulations to rats (see Example 8).

The invention will now be described by reference to the following examples which are merely illustrative and are not to be construed as a limitation of the scope of the present invention.

### EXAMPLES

### General description of methods

### Administration into mid-jejunum in rats:

After checking of the depth of anesthesia the animal was placed on its back and a 3 - 5 cm long midline incision was made in the skin of abdomen. The wound margins were released from the base. The muscle layer of the abdominal wall was carefully cut in the middle line (linea alba).

The caecum was exposed and the small intestine was gradually pulled out of the abdominal cavity and the position of the spot convenient for introduction of catheter was measured using a PE tubing with marks at a distance of 40, 50, and 60 cm. Pulling the intestine was performed very carefully to avoid injury of blood vessels and mesentery. The intestine was penetrated by the catheter tip and the catheter was inserted downstream into the jejunal lumen at a distance of 50 (35-65) cm from caecum in a spot without feces, outside the area with accumulated lymphatic tissue and outside the blood vessels and fixed with ligature. The distance of the spot from caecum was measured and recorded.

The pulled loops of small intestine were replaced into the abdominal cavity, 2 ml of sterile saline were flushed over the intestine and the abdominal cavity was closed with metal wound clips in two layers. The prepared syringes filled with the formulations were gradually attached to the inserted catheters. Dosing was performed slowly. The syringes with peptide or protein drug were attached to the inserted cannula until the end of the experiment.

Correctness of the application was checked. The abdominal cavity was opened by cutting next to the surgical wound to see if the catheter was still in place. About 1 ml of air was flushed into the lumen of the intestine with a syringe to reveal possible penetration of intestinal wall. The site of application was inspected and possible changes were identified.

### Example 1: Compatibility of absorption enhancers with the trace element copper

Various absorption enhancers were screened regarding their compatibility with the trace element copper in the two main oxidation states as Cu²⁺ and Cu⁺. The results of this test are shown in the following Table 1:

**Table 1: Compatibility of various absorption enhancers with Cu²⁺ and Cu⁺ salts.**

| **Absorption enhancer (10 mg/ml)** | **Copper salt (1 mg/ml)** | **Reducing agent (10 mg/ml)** | **Dissolution in aqueous medium (2 ml)** |
|---|---|---|---|
| Sodium caprate | CuSO₄ | Sodium ascorbate | Precipitation |
| Sodium caprylate | CuSO₄ | Sodium ascorbate | Precipitation |
| Sodium lauroyl sarcosinate | CuSO₄ | Sodium ascorbate | Precipitation |
| Lauroyl carnitine | CuSO₄ | Sodium ascorbate | Clear solution |
| Lauroyl carnitine | CuSO₄ | Glutathione | Clear solution |
| Sodium dodecyl sulfate | CuSO₄ | Sodium ascorbate | Clear solution |
| Multitrope 1620 LQ (alkylsaccharide) | CuSO₄ | Sodium ascorbate | Clear solution |
| n-Octyl-beta-D-glucopyranoside | CuSO₄ | Sodium ascorbate | Clear solution |
| n-Dodecyl-beta-D-maltoside | CuSO₄ | Sodium ascorbate | Clear solution |
| Cremophor EL | CuSO₄ | Sodium ascorbate | Clear solution |
| Sodium salicylate | CuSO₄ | Sodium ascorbate | Clear solution |
| EDTA | CuSO₄ | Sodium ascorbate | Clear solution |
| Sodium citrate | CuSO₄ | Sodium ascorbate | Clear solution |
| Methyl-betacyclodextrin | CuSO₄ | Sodium ascorbate | Clear solution |
| Sodium caprylate | Copper (I) acetate | Sodium ascorbate | Clear solution |
| Sodium caprate | Copper (I) acetate | Sodium ascorbate | Clear solution |
| Sodium caprate | Cu(I)Cl | Sodium ascorbate | Clear solution |
| Sodium caprylate | Cu(I)Cl | Sodium ascorbate | Clear solution |

The term clear solution as used in this table refers to that no clear visible precipitation or flocculation has been observed. The term clear solution also includes slightly colored clear solutions such as yellowish or orange solutions.

Conclusion: Certain absorption enhancers such as medium chain fatty acid salts and derivatives are not well compatible with divalent copper salts. However, zwitter-ionic as well as non-ionic surfactants showed good compatibility with copper salts. Moreover, monovalent copper also showed compatibility with sodium caprate and caprylate. A disadvantage of monovalent copper salts is their low aqueous solubility and the instability of the Cu⁺ oxidation state in aqueous solutions. The monovalent copper salt Cu(I)Cl, on the other hand, has aqueous solubility and oxidation state stability.

### Example 2: The pharmacodynamic and pharmacokinetic profiles of human insulin formulations after administration into mid-jejunum of Sprague Dawley rats (reference)

Method - Administration into rat jejunum: The direct application of peptide or protein drugs and their formulations into mid-jejunum (the middle part of the small intestine - jejunum) enables to study their ability to pass through the intestinal barrier and to resist the degradation caused by the digestive enzymes. Sprague-Dawley rats, males, weighing 250 - 300 g were used. The studies were preformed under anesthesia. The results obtained are indicated in the following Table 2:

**Table 2: Pharmacokinetic profiles of human insulin formulations.**

| **Human insulin** | **Absorption enhancer (10 mg/ml)** | **Trace element (1 mg/ml)** | **Reducing agent** | **AUC₍₀₋ₜ₎ (pmol/l x min)** | **Cₘₐₓ (pmol/l)** |
|---|---|---|---|---|---|
| 22 IU/kg | Sodium caprate | - | - | 2093±1759 | 28±24 |
| 22 IU/kg | Sodium caprate | CuSO₄ | Ascorbate | 2505±2921 | 37±54 |

Conclusion: A simple physical blend of human insulin and the classical absorption enhancer sodium caprate in the presence of the trace element copper with ascorbate as a reducing agent moderately improved the oral bioavailability of human insulin. A more pronounced improvement of the oral bioavailability was not observed in this experiment because of the poor aqueous solubility of both sodium caprate and insulin in the presence of 1 mg/ml Cu²⁺.

### Example 3: The pharmacodynamic and pharmacokinetic profiles of human insulin formulations after administration into mid-jejunum of Sprague Dawley rats (reference)

In order to improve insulin solubility, Cu²⁺ and insulin were dosed separately. Experimental design - 10 minutes predosing of the trace element copper with sodium ascorbate followed by dosing of an insulin solution comprising one of various absorption enhancers. In such a setup, insulin was found to retain good aqueous solubility.

Predosing solution: 1 mg/ml CuSO₄ and 10 mg/ml sodium ascorbate

The respective insulin solutions: 22 IU/kg human insulin, and absorption enhancer
a) 22 IU/kg human insulin and 50 mg/ml sodium caprate
b) 22 IU/kg human insulin and 100 mg/ml Cremophor EL and 10 mg/ml Na₂HPO₄
c) 22 IU/kg human insulin and 50 mg/ml lauroyl-DL-carnitine-chloride and 40 mg/ml Na₂HPO₄

**Table 3: Pharmacokinetic profiles of human insulin formulations comprising different absorption enhancers after pre-dosing of copper sulfate and sodium ascorbate.**

| **Predosing solution** | **Human insulin** | **Absorption enhancer** | **Buffer agent** | **AUC₍₀₋ₜ₎ (pmol/l x min)** | **Cₘₐₓ (pmol/l)** |
|---|---|---|---|---|---|
| CuSO₄ and ascorbate | 22 IU/kg | Sodium caprate | - | 5351 ± 2289 | 72 ± 26 |
| CuSO₄ and ascorbate | 22 IU/kg | Cremophor EL | Na₂HPO₄ | 2340 ± 899 | 48 ± 23 |
| CuSO₄ and ascorbate | 22 IU/kg | Lauroyl carnitine chloride | Na₂HPO₄ | 145896 ± 89530 | 1850 ± 1117 |

Conclusion: The results show that compositions comprising the zwitter-ionic absorption enhancer lauroyl-carnitine resulted in 27 to 62-fold improved insulin absorption (AUC₍ₒ₋ₜ₎) as compared with compositions comprising absorption enhancers such as sodium caprate or Cremophor EL, respectively. Moreover, the predosing of CuSO₄ and sodium ascorbate prior to the administration of insulin and sodium caprate was found to provide a more favorable insulin absorption than the simultaneous administration of these agents, as tested in Example 2.

### Example 4: Pharmacodynamics in non human primates after oral administration of solid oral compositions of human insulin (reference)

Composition A:
Double capsule formulations providing a physical barrier between insulin and Cu²⁺ and the reducing agent sodium ascorbate are described. A small size 4 capsule is placed into a larger size 1 capsule. In such a solid oral dosage form the therapeutic polypeptide drug is not in intimate contact with the trace element copper and the reducing agent. Further, copper and ascorbate will be released *in vivo* prior to insulin. Such a composition will also improve insulin solubility.

The composition comprises 4 mg human insulin and 50 mg sodium salicylate in size 4 hard capsule and 100 mg sodium ascorbate, 1 mg CuSO₄ and 100 mg sodium salicylate in size 1 capsule. The size 4 capsule is placed into the size 1 capsule. The capsule was dosed per oral to a female cynomolgus monkey with additional 5 ml of tap water.

As control, a capsule comprising 4 mg of human insulin and 200 mg of the known absorption enhancer sodium caprylate was used.

**Table 4: % Glucose reduction in non human primate after oral administration of composition A**

| 4 mg Insulin | 0 min | 20 min | 40 min | 60 min | 120 min | 150 min |
|---|---|---|---|---|---|---|
| Control: 200 mg sodium caprylate | 0 | +16 | +20 | +47 | 1 | 0 |
| 150 mg sodium salicylate, 100 mg sodium ascorbate, 1 mg CuSO₄ | 0 | +59 | -14 | -19 | -14 | -1 |

Conclusion: A solid oral insulin composition comprising the absorption enhancer sodium salicylate in the presence of the trace element copper and the reducing agent sodium ascorbate resulted in a clear pharmacodynamic effect (reduction of the blood glucose levels). The control formulation did not show any reduction of the blood glucose levels.

Composition B:
Double capsule: Double capsule formulations providing a physical barrier between insulin and Cu²⁺ and the reducing agent sodium ascorbate are described. A small size 4 capsule is placed into a larger size 1 capsule. In such a solid oral dosage form the therapeutic polypeptide drug is not in intimate contact with the trace element copper and the reducing agent. Further, copper and ascorbate will be released *in vivo* prior to insulin.

The composition comprises 4 mg human insulin and 200 mg Multitrope 1620 LQ-(MV) which is a C8 and C10 fatty acid acylated polysaccharide and 5 mg sodium citrate in size 4 hard capsule and 180 mg sodium ascorbate, 0.5 mg CuSO₄ in size 1 hard capsule. The size 4 capsule is placed into the size 1 capsule. In addition, 5 ml of TRIS (4 mg/ml) were dosed per oral to a female cynomolgus monkey.

**Table 5: % Glucose reduction in non human primate after oral administration of composition B**

| 4 mg Insulin | 0 min | 20 min | 40 min | 60 min | 90 min | 120 min | 150 min |
|---|---|---|---|---|---|---|---|
| Control: 200 mg sodium caprylate | 0 | +16 | +20 | +47 | +16 | 1 | 0 |
| 200 mg Multitrope 1620 LQ-(MV), 180 mg sodium ascorbate, 0.5 mg CuSO₄, 5 mg sodium citrate | 0 | -33 | -21 | -12 | -15 | -15 | -19 |

Conclusion: An oral insulin composition comprising the non-ionic absorption enhancer Multitrope 1620 LQ which is an alkylsaccharide in the presence of the trace element copper and the reducing agent sodium ascorbate resulted in a substantial pharmacodynamic effect (reduction of the blood glucose levels).

### Example 5: Pharmacokinetic profile of the glycopeptide vancomycin after administration into ileum of Sprague Dawley rats

Vancomycin hydrochloride is a glycopeptide antibiotic with an negligible oral bioavailability in the 0.1% range but usually under detection limit.
a) Vancomycin was administered i.v. in dosing volume of 1 ml/kg (final concentration of 5 mg/ml) to anesthetized rats.
b) Vancomycin was dosed into ileum in volume of 0.4 ml/kg (final concentration of 50 mg/ml) to anesthetized rats.

The vancomycin plasma concentrations were determined on biochemical autoanalyser Hitachi 912 using commercial vancomycin kit VANC2 (Roche Diagnostics GmbH, Germany). The absolute bioavailability (F) was calculated using equation F (%) = (AUCᵢₗₑᵤₘ/AUC_{i.v.}) x (Dose_{i.v.}/Doseᵢₗₑᵤₘ) x 100.

**Table 6: Pharmacokinetic profile of vancomycin formulation.**

| **Vancomycin** | **Lauroyl carnitine** | **Trace element CuSO₄** | **Reducing agent sodium ascorbate** | **AUC₍₀₋ₜ₎ (µg/ml x min)** | **Cₘₐₓ (µg/ml)** | **F (%)** |
|---|---|---|---|---|---|---|
| 5 mg/kg i.v. | - | - | - | 662 ± 60 | 8,8 ± 0,3 | 100 ± 7 |
| 20 mg/kg ileum | 50 mg/ml | 1 mg/ml | 250 mg/ml | 180 ± 45 | 2,4 ± 0,2 | 8 ± 0,4 |

Conclusion: An oral vancomycin formulation according to the invention increased markedly its bioavailability in comparison with published F values after oral administration (Prasad YV et al., Int J Pharm. 2003, 250(1):181-90; Van Bambeke F, Curr Opin Investig Drugs. 2006, 7(8):740-9).

### Example 6: Pharmacokinetic profiles of liraglutide after administration into mid-jejunum of Sprague Dawley rats

2.4 mg/kg of the polypeptide liraglutide were dosed into the mid-jejunum of anesthetized Sprague Dawley rats in the presence of the zwitter-ionic absorption enhancer lauroyl carnitine chloride ("LCC") (20 mg/ml) with and without the addition of (i) the trace element CuSO₄ (1 mg/ml) and the reducing agent sodium ascorbate (40 mg/ml) or (ii) the trace element ZnSO₄ (1 mg/ml) and the reducing agent glutathione (40 mg/ml). All formulations further comprised Na₂HPO₄ to result in a neutral pH between 7 to 8. The results of these experiments are shown in Figure 1.

Conclusion: The combination of the zwitter-ionic absorption enhancer lauroyl carnitine (LCC) in the presence of the trace element copper and the reducing agent sodium ascorbate substantially increased the intestinal absorption of the polypeptide drug liraglutide. The reducing agent glutathione (GSH) in the presence of Zn²⁺ also showed improved liraglutide absorption albeit less pronounced than the composition comprising Cu²⁺.

### Example 7: Pharmacokinetic profiles of liraglutide after administration into mid-jejunum of Sprague Dawley rats

2.4 mg/kg of the polypeptide liraglutide were dosed into the mid-jejunum of anesthetized Sprague Dawley rats in the presence of the anionic absorption enhancer sodium dodecyl sulfate (SDS) (20 mg/ml) and the trace element CuSO₄(1 mg/ml) with and without the reducing agent reduced glutathione (40 mg/ml).

**Table 7: Pharmacokinetic profiles of liraglutide formulations.**

| **Liraglutide** | **Enhancer: Sodium dodecyl sulfate** | **Trace element: CuSO₄** | **Reducing agent: Reduced glutathione (GSH)** | **AUC₍₀₋ₜ₎ (µg/ml x min)** | **Cₘₐₓ (ng/ml)** |
|---|---|---|---|---|---|
| 2.4 mg/kg into jejunum | 20 mg/ml | 1 mg/ml | - | 5504 ± 5729 | 32 |
| 2.4 mg/kg into jejunum | 20 mg/ml | 1 mg/ml | 40 mg/ml | 49090 ± 23073 | 416 |

Conclusion: The combination of an absorption enhancer in the presence of the trace element copper and the reducing agent glutathione substantially increased the intestinal absorption of the polypeptide drug liraglutide.

### Example 8: Pharmacodynamic profiles after insulin and liraglutide administration into mid-jejunum of Sprague Dawley rats

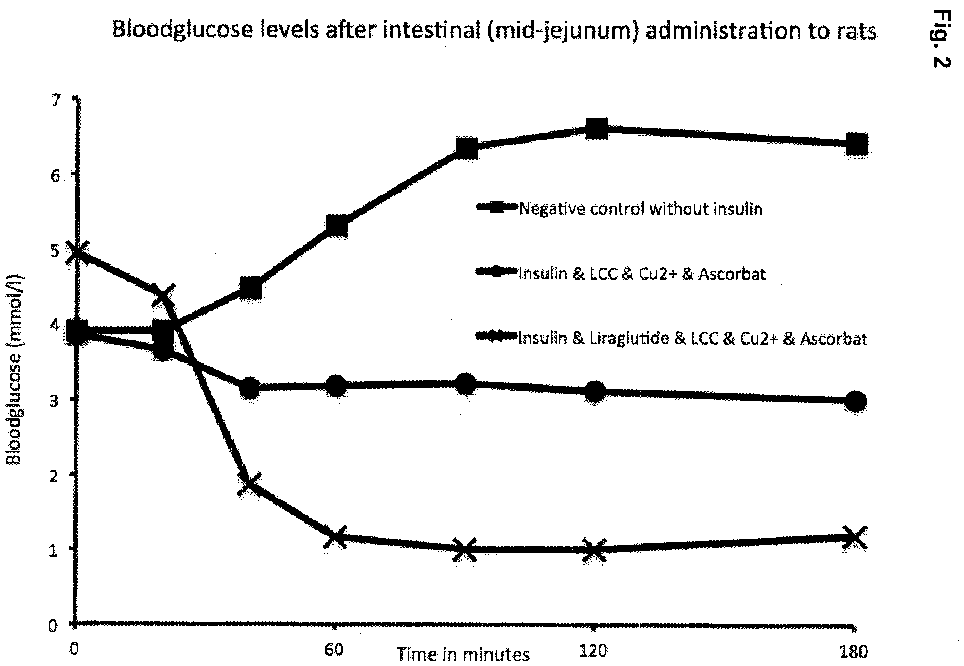

A combination of human insulin (22 IU/kg) and liraglutide (2.4 mg/kg) was dosed together into the mid-jejunum of Sprague Dawley rats in the presence of the absorption enhancer lauroyl carnitine chloride ("LCC") (50 mg/ml) and Na₂HPO₄ (40 mg/ml). A solution of the trace element copper as CuSO₄ (1 mg/ml) and sodium ascorbate (10 mg/ml) was dosed 10 minutes prior to insulin and liraglutide into the mid-jejunum of rats. The results of this experiment are shown in Figure 2.

Conclusion: A combination of insulin and the GLP-1 receptor agonist liraglutide in the presence of lauroyl carnitine after predosing of Cu²⁺ and sodium ascorbate resulted in a substantial reduction of the blood glucose levels.

### Example 9: Pharmacokinetic profile of the somatostatin analog octreotide after administration into ileum of Sprague Dawley rats

Octreotide is an octapeptide with very low oral bioavailability of less than 1%.
a) Octreotide was administered s.c. at a concentration of 1 mg/kg body weight to anesthetized rats (n=3).
b) Octreotide was dosed into distal small intestine (ileum) at a concentration of 0.92 mg/kg body weight to anesthetized rats (n=8). TRIS buffer was used to generate a neutral pH.

The octreotide plasma concentrations were determined using a commercial octreotide kit (AB Biolabs, USA, cat. number CEK 0110-01).

**Table 8: Pharmacokinetic profiles of octreotide formulations.**

| **Octreotide** | **Enhancer:** Lauroyl carnitine chloride | **Trace element:** CuSO₄ | **Reducing agent:** Sodium ascorbate | **Buffering agent:** TRIS | **AUC₍₀₋₉₀₎** (ng/ml x min) | **Cₘₐₓ** (ng/ml) | **F (%)** |
|---|---|---|---|---|---|---|---|
| 1 mg/kg s.c. (n=3) | - | - | - | - | 10045 ± 1837 | 153.7 ± 29.8 | 100 ± 18.3 |
| 0.92 mg/kg ileum (n=8) | 30 mg/ml | 1 mg/ml | 30 mg/ml | 22.5 mg/ml | 1504 ± 725 | 25.8 ± 14.2 | 16.3 ± 7.8 |

Conclusion: An oral octreotide formulation according to the invention, comprising a copper(II) salt, sodium ascorbate as reducing agent and lauroyl carnitine chloride as an exemplary absorption enhancer, showed a markedly increased bioavailability.

## Claims

1. A peptide or protein drug for use as a medicament, wherein said peptide or protein drug is to be administered orally in combination with:
a pharmaceutically acceptable copper salt/complex and/or a pharmaceutically acceptable zinc salt/complex; and
a pharmaceutically acceptable reducing agent;
wherein the peptide or protein drug is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog,
pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

2. A pharmaceutically acceptable copper salt/complex for use in therapy, wherein said copper salt/complex is to be administered orally in combination with:
a pharmaceutically acceptable reducing agent; and
a peptide or protein drug which is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

3. A pharmaceutically acceptable zinc salt/complex for use in therapy, wherein said zinc salt/complex is to be administered orally in combination with:
a pharmaceutically acceptable reducing agent; and
a peptide or protein drug which is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

4. A pharmaceutically acceptable reducing agent for use in therapy, wherein said reducing agent is to be administered orally in combination with:
a pharmaceutically acceptable copper salt/complex and/or a pharmaceutically acceptable zinc salt/complex; and
a peptide or protein drug which is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

5. A pharmaceutical composition for use in therapy by oral administration, comprising:
a peptide or protein drug;
a pharmaceutically acceptable copper salt/complex and/or a pharmaceutically acceptable zinc salt/complex; and
a pharmaceutically acceptable reducing agent;
wherein the peptide or protein drug is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

6. A pharmaceutical dosage form for use in therapy by oral administration, comprising:
a peptide or protein drug;
a pharmaceutically acceptable copper salt/complex and/or a pharmaceutically acceptable zinc salt/complex; and
a pharmaceutically acceptable reducing agent;
wherein the peptide or protein drug is physically separated from the pharmaceutically acceptable copper salt/complex and the pharmaceutically acceptable zinc salt/complex within the pharmaceutical dosage form;
wherein the peptide or protein drug is:
(i) a peptide or protein drug having a molecular weight of about 2 kDa to about 5 kDa, which is not glucagon; or
(ii) a peptide or protein drug having a molecular weight of equal to or less than about 50 kDa, which is selected from GLP-1, a GLP-1 analog, an acylated GLP-1 analog, a diacylated GLP-1 analog, semaglutide, liraglutide, exenatide, lixizenatide, a dual agonist of the GLP-1 receptor and the glucagon receptor, amylin, an amylin analog, pramlintide, a somatostatin analog, octreotide, lanreotide, pasireotide, goserelin, buserelin, leptin, a leptin analog, metreleptin, peptide YY, a peptide YY analog, glatiramer, leuprolide, teriparatide, desmopressin, a glycopeptide antibiotic, a glycosylated cyclic or polycyclic nonribosomal peptide antibiotic, vancomycin, teicoplanin, telavancin, bleomycin, ramoplanin, decaplanin, bortezomib, cosyntropin, chorionic gonadotropin, menotropin, sermorelin, luteinizing-hormone-releasing hormone, calcitonin, calcitonin-salmon, pentagastrin, oxytocin, neseritide, anakinra, enfuvirtide, pegvisomant, dornase alfa, lepirudin, anidulafungin, eptifibatide, interferon alfacon-1, interferon beta-1a, interferon beta-1b, peginterferon alfa-2a, peginterferon alfa-2b, peginterferon beta-1a, fibrinolysin, vasopressin, aldesleukin, darbepoetin alfa, filgrastim, interleukin-11, cyclosporine, urokinase, viomycin, thyrotropin-releasing hormone, leucine-enkephalin, methionine-enkephalin, substance P, adrenocorticotropic hormone, parathyroid hormone, and pharmaceutically acceptable salts thereof.

7. The peptide or protein drug for use according to claim 1 or the copper salt/complex for use according to claim 2 or the reducing agent for use according to claim 4 or the pharmaceutical composition for use according to claim 5 or the pharmaceutical dosage form for use according to claim 6, wherein said copper salt/complex is a copper(II) salt/complex selected from copper sulfate, copper carbonate, copper(II) lysine complex, copper(II) citrate, and copper(II) gluconate, or said copper salt/complex is a copper(I) salt/complex selected from copper(I) chloride and copper(I) acetate.

8. The peptide or protein drug for use according to claim 1 or the zinc salt/complex for use according to claim 3 or the reducing agent for use according to claim 4 or the pharmaceutical composition for use according to claim 5 or the pharmaceutical dosage form for use according to claim 6, wherein said zinc salt/complex is a zinc(II) salt/complex selected from zinc sulfate, zinc chloride, zinc acetate, zinc oxide, zinc ascorbate, zinc caprylate, zinc gluconate, zinc stearate, and zinc carbonate.

9. The peptide or protein drug for use according to any one of claims 1, 7 or 8 or the copper salt/complex for use according to claim 2 or 7 or the zinc salt/complex for use according to claim 3 or 8 or the reducing agent for use according to any one of claims 4, 7 or 8 or the pharmaceutical composition for use according to any one of claims 5, 7 or 8 or the pharmaceutical dosage form for use according to any one of claims 6 to 8, wherein said reducing agent is selected from ascorbic acid, reduced glutathione, cysteine, uric acid, a reducing sugar, glucose, glyceraldehyde, galactose, lactose, maltose, mannitol, α-tocopherol, vitamin A, α-lipoic acid, dihydro-α-lipoic acid, a thiol-bearing compound, a thiomer, and pharmaceutically acceptable salts thereof.

10. The peptide or protein drug for use according to any one of claims 1 or 7 to 9 or the copper salt/complex for use according to any one of claims 2, 7 or 9 or the zinc salt/complex for use according to any one of claims 3, 8 or 9 or the reducing agent for use according to any one of claims 4 or 7 to 9 or the pharmaceutical composition for use according to any one of claims 5 or 7 to 9 or the pharmaceutical dosage form for use according to any one of claims 6 to 9, wherein said peptide or protein drug or said copper salt/complex or said zinc salt/complex or said reducing agent is to be administered orally in combination with an absorption enhancer, or wherein said pharmaceutical composition or said pharmaceutical dosage form further comprises an absorption enhancer.

11. The peptide or protein drug for use according to claim 10 or the copper salt/complex for use according to claim 10 or the zinc salt/complex for use according to claim 10 or the reducing agent for use according to claim 10 or the pharmaceutical composition for use according to claim 10 or the pharmaceutical dosage form for use according to claim 10, wherein said absorption enhancer is selected from C₈₋₂₀ alkanoyl carnitine, salicylic acid, a salicylic acid derivative, 3-methoxysalicylic acid, 5-methoxysalicylic acid, homovanillic acid, a C₈₋₂₀ alkanoic acid, citric acid, a fatty acid acylated amino acid, a C₈₋₂₀ alkanoyl sarcosinate, an alkylsaccharide, a C₈₋₁₀ alkylpolysaccharide, n-octyl-beta-D-glucopyranoside, n-dodecyl-beta-D-maltoside, a cyclodextrine, α-cydodextrin, β-cyclodextrin, γ-cyclodextrin, methyl-β-cyclodextrin, hydroxypropyl β-cyclodextrin, sulfobutylether β-cydodextrin, sodium N-[8-(2-hydroxybenzoyl)amino]caprylate, a thiomer, a calcium chelating compound, ethylenediaminetetraacetic acid, ethylene glycol tetraacetic acid, polyacrylic acid, cremophor EL, chitosan, N,N,N-trimethyl chitosan, benzalkonium chloride, bestatin, cetylpyridinium chloride, cetyltrimethylammonium bromide, a C₂₋₂₀ alkanol, a C₈₋₂₀ alkenol, a C₈₋₂₀ alkenoic acid, dextran sulfate, diethyleneglycol monoethyl ether, 1-dodecylazacyclo-heptan-2-one, ethyl caprylate, glyceryl monolaurate, lysophosphatidylcholine, menthol, a C₈₋₂₀ alkylamine, a C₈₋₂₀ alkenylamine, phosphatidylcholine, a poloxamer, polyethylene glycol monolaurate, polyoxyethylene, polypropylene glycol monolaurate, a polysorbate, a deoxycholate, sodium glycocholate, sodium glycodeoxycholate, sodium lauryl sulfate, a taurocholate, a taurodeoxycholate, sucrose laurate, a sulfoxide, decyl methyl sulfoxide, dimethyl sulfoxide, cyclopentadecalactone, 8-(N-2-hydroxy-5-chloro-benzoyl)-amino-caprylic acid, dodecyl-2-N,N-dimethylamino propionate, D-α-tocopheryl polyethylene glycol-1000 succinate, and pharmaceutically acceptable salts thereof.

12. The peptide or protein drug for use according to claim 11 or the copper salt/complex for use according to claim 11 or the zinc salt/complex for use according to claim 11 or the reducing agent for use according to claim 11 or the pharmaceutical composition for use according to claim 11 or the pharmaceutical dosage form for use according to claim 11, wherein said absorption enhancer is a fatty acid acylated amino acid selected from sodium lauroyl alaninate, N-dodecanoyl-L-alanine, sodium lauroyl asparaginate, N-dodecanoyl-L-asparagine, sodium lauroyl aspartic acid, N-dodecanoyl-L-aspartic acid, sodium lauroyl cysteinate, N-dodecanoyl-L-cysteine, sodium lauroyl glutamic acid, N-dodecanoyl-L-glutamic acid, sodium lauroyl glutaminate, N-dodecanoyl-L-glutamine, sodium lauroyl glycinate, N-dodecanoyl-L-glycine, sodium lauroyl histidinate, N-dodecanoyl-L-histidine, sodium lauroyl isoleucinate, N-dodecanoyl-L-isoleucine, sodium lauroyl leucinate, N-dodecanoyl-L-leucine, sodium lauroyl methioninate, N-dodecanoyl-L-methionine, sodium lauroyl phenylalaninate, N-dodecanoyl-L-phenylalanine, sodium lauroyl prolinate, N-dodecanoyl-L-proline, sodium lauroyl serinate, N-dodecanoyl-L-serine, sodium lauroyl threoninate, N-dodecanoyl-L-threonine, sodium lauroyl tryptophanate, N-dodecanoyl-L-tryptophane, sodium lauroyl tyrosinate, N-dodecanoyl-L-tyrosine, sodium lauroyl valinate, N-dodecanoyl-L-valine, sodium lauroyl sarcosinate, N-dodecanoyl-L-sarcosine, sodium capric alaninate, N-decanoyl-L-alanine, sodium capric asparaginate, N-decanoyl-L-asparagine, sodium capric aspartic acid, N-decanoyl-L-aspartic acid, sodium capric cysteinate, N-decanoyl-L-cysteine, sodium capric glutamic acid, N-decanoyl-L-glutamic acid, sodium capric glutaminate, N-decanoyl-L-glutamine, sodium capric glycinate, N-decanoyl-L-glycine, sodium capric histidinate, N-decanoyl-L-histidine, sodium capric isoleucinate, N-decanoyl-L-isoleucine, sodium capric leucinate, N-decanoyl-L-leucine, sodium capric methioninate, N-decanoyl-L-methionine, sodium capric phenylalaninate, N-decanoyl-L-phenylalanine, sodium capric prolinate, N-decanoyl-L-proline, sodium capric serinate, N-decanoyl-L-serine, sodium capric threoninate, N-decanoyl-L-threonine, sodium capric tryptophanate, N-decanoyl-L-tryptophane, sodium capric tyrosinate, N-decanoyl-L-tyrosine, sodium capric valinate, N-decanoyl-L-valine, sodium capric sarcosinate, N-decanoyl-L-sarcosine, sodium oleoyl sarcosinate, sodium N-decylleucine, sodium stearoyl glutamate, sodium myristoyl glutamate, sodium lauroyl glutamate, sodium cocoyl glutamate, sodium cocoyl glycinate, sodium N-decyl leucine, sodium cocoyl glycine, sodium cocoyl glutamate, sodium lauroyl alaninate, N-dodecanoyl-L-alanine, sodium lauroyl asparaginate, N-dodecanoyl-L-asparagine, sodium lauroyl aspartic acid, N-dodecanoyl-L-aspartic acid, sodium lauroyl cysteinate, N-dodecanoyl-L-cysteine, sodium lauroyl glutamic acid, N-dodecanoyl-L-glutamic acid, sodium lauroyl glutaminate, N-dodecanoyl-L-glutamine, sodium lauroyl glycinate, N-dodecanoyl-L-glycine, sodium lauroyl histidinate, N-dodecanoyl-L-histidine, sodium lauroyl isoleucinate, N-dodecanoyl-L-isoleucine, sodium lauroyl leucinate, N-dodecanoyl-L-leucine, sodium lauroyl methinoninate, N-dodecanoyl-L-methionine, sodium lauroyl phenylalaninate, N-dodecanoyl-L-phenylalanine, sodium lauroyl prolinate, N-dodecanoyl-L-proline, sodium lauroyl serinate, N-dodecanoyl-L-serine, sodium lauroyl threoninate, N-dodecanoyl-L-threonine, sodium lauroyl tryptophanate, N-dodecanoyl-L-tryptophane, sodium lauroyl tyrosinate, N-dodecanoyl-L-tyrosine, sodium lauroyl valinate, N-dodecanoyl-L-valine, N-dodecanoyl-L-sarcosine, sodium capric alaninate, N-decanoyl-L-alanine, sodium capric asparaginate, N-decanoyl-L-asparagine, sodium capric aspartic acid, N-decanoyl-L-aspartic acid, sodium capric cysteinate, N-decanoyl-L-cysteine, sodium capric glutamic acid, N-decanoyl-L-glutamic acid, sodium capric glutaminate, N-decanoyl-L-glutamine, sodium capric glycinate, N-decanoyl-L-glycine, sodium capric histidinate, N-decanoyl-L-histidine, sodium capric isoleucinate, N-decanoyl-L-isoleucine, sodium capric leucinate, N-decanoyl-L-leucine, sodium capric methioninate, N-decanoyl-L-methionine, sodium capric phenylalaninate, N-decanoyl-L-phenylalanine, sodium capric prolinate, N-decanoyl-L-proline, sodium capric serinate, N-decanoyl-L-serine, sodium capric threoninate, N-decanoyl-L-threonine, sodium capric tryptophanate, N-decanoyl-L-tryptophane, sodium capric tyrosinate, N-decanoyl-L-tyrosine, sodium capric valinate, N-decanoyl-L-valine, sodium capric sarcosinate, sodium oleoyl sarcosinate, and pharmaceutically acceptable salts thereof.

13. The pharmaceutical composition for use according to any one of claims 10 to 12 or the pharmaceutical dosage form for use according to any one of claims 10 to 12, wherein said pharmaceutical composition or said pharmaceutical dosage form comprises:
the copper salt/complex in an amount of about 0.1 mg to about 5 mg per dosage unit,
and/or the zinc salt/complex in an amount of about 0.1 mg to about 50 mg per dosage unit;
the reducing agent in an amount of about 1 mg to about 1000 mg per dosage unit; and
the absorption enhancer in an amount of about 10 mg to about 1000 mg per dosage unit.

## Patentansprüche

1. Peptid- oder Proteinarzneistoff zur Verwendung als Medikament, wobei der Peptid- oder Proteinarzneistoff oral in Kombination mit:
einem pharmazeutisch verträglichen Kupfersalz-Komplex und/oder einem pharmazeutisch verträglichen Zinksalz-Komplex; und
einem pharmazeutisch verträglichen Reduktionsmittel zu verabreichen ist:
wobei der Peptid- oder Proteinarzneistoff:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixizenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Calcitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon-alfa-2b, Peginterferon-beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

2. Pharmazeutisch verträglicher Kupfersalz-Komplex zur therapeutischen Verwendung, wobei der Kupfersalz-Komplex oral in Kombination mit:
einem pharmazeutisch verträglichen Reduktionsmittel; und
einem Peptid- oder Proteinarzneistoff zu verabreichen ist, der:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixizenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Calcitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon-alfa-2b, Peginterferon-beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

3. Pharmazeutisch verträglicher Zinksalz-Komplex zur therapeutischen Verwendung, wobei der Zinksalz-Komplex oral in Kombination mit:
einem pharmazeutisch verträglichen Reduktionsmittel; und
einem Peptid- oder Proteinarzneistoff
zu verabreichen ist, der:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixizenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Calcitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon-alfa-2b, Peginterferon-beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

4. Pharmazeutisch verträgliches Reduktionsmittel zur therapeutischen Verwendung, wobei das Reduktionsmittel oral in Kombination mit:
einem pharmazeutisch verträglichen Kupfersalz-Komplex und/oder einem pharmazeutisch verträglichen Zinksalz-Komplex; und
einem Peptid- oder Proteinarzneistoff
zu verabreichen ist, der:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixizenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Calcitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon-alfa-2b, Peginterferon-beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

5. Arzneimittel, zur therapeutischen Verwendung durch orale Verabreichung umfassend:
einen Peptid- oder Proteinarzneistoff;
einen pharmazeutisch verträglichen Kupfersalz-Komplex und/oder einen pharmazeutisch verträglichen Zinksalz-Komplex; und
ein pharmazeutisch verträgliches Reduktionsmittel;
wobei der Peptid- oder Proteinarzneistoff:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixizenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Calcitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon-alfa-2b, Peginterferon-beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

6. Pharmazeutische Darreichungsform zur therapeutischen Verwendung durch orale Verabreichung, umfassend:
einen Peptid- oder Proteinarzneistoff;
einen pharmazeutisch verträglichen Kupfersalz-Komplex und/oder einen pharmazeutisch verträglichen Zinksalz-Komplex; und
ein pharmazeutisch verträgliches Reduktionsmittel;
wobei der Peptid- oder Proteinarzneistoff innerhalb der pharmazeutischen Darreichungsform von dem pharmazeutisch verträglichen Kupfersalz-Komplex und/oder dem pharmazeutisch verträglichen Zinksalz-Komplex physisch getrennt ist;
wobei der Peptid- oder Proteinarzneistoff:
(i) ein Peptid- oder Proteinarzneistoff ist, der ein Molekulargewicht von etwa 2 kDa bis etwa 5 kDa hat, der nicht Glucagon ist; oder
(ii) ein Peptid- oder Proteinarzneistoff ist, der ein Molekulargewicht von etwa 50 kDa oder weniger hat, der ausgewählt ist aus GLP-1, einem GLP-1-Analog, einem acylierten GLP-1-Analog, einem diacylierten GLP-1-Analog, Semaglutid, Liraglutid, Exenatid, Lixisenatid, einem dualen Agonisten des GLP-1-Rezeptors und des Glucagon-Rezeptors, Amylin, einem Amylin-Analog, Pramlintid, einem Somatostatin-Analog, Octreotid, Lanreotid, Pasireotid, Goserelin, Buserelin, Leptin, einem Leptin-Analog, Metreleptin, Peptid YY, einem Peptid-YY-Analog, Glatiramer, Leuprolid, Teriparatid, Desmopressin, einem Glycopeptid-Antibiotikum, einem glycosylierten cyclischen oder polycyclischen nicht-ribosomalem Peptid-Antibiotikum, Vancomycin, Teicoplanin, Telavancin, Bleomycin, Ramoplanin, Decaplanin, Bortezomib, Cosyntropin, Choriongonadotropin, Menotropin, Sermorelin, Luteinisierendes Hormon-freisetzendem Hormon, Calcitonin, Lachs-Clacitonin, Pentagastrin, Oxytocin, Neseritid, Anakinra, Enfuvirtid, Pegvisomant, Dornase alfa, Lepirudin, Anidulafungin, Eptifibatid, Interferon alfacon-1, Interferon beta-1a, Interferon beta-1b, Peginterferon alfa-2a, Peginterferon alfa-2b, Peginterferon beta-1a, Fibrinolysin, Vasopressin, Aldesleukin, Darbepoetin alfa, Filgrastim, Interleukin-11, Cyclosporin, Urokinase, Viomycin, Thyrotropin-freisetzendem Hormon, Leucin-Enkephalin, Methionin-Enkephalin, Substanz P, Adrenocorticotropem Hormon, Parathyroid-Hormon und pharmazeutisch verträglichen Salzen davon.

7. Peptid- oder Proteinarzneistoff zur Verwendung nach Anspruch 1 oder Kupfersalz-Komplex zur Verwendung nach Anspruch 2 oder Reduktionsmittel zur Verwendung nach Anspruch 4 oder Arzneimittel zur Verwendung nach Anspruch 5 oder pharmazeutische Darreichungsform zur Verwendung nach Anspruch 6, wobei der Kupfersalz-Komplex ein Kupfer(II)-Salz-Komplex ist, der ausgewählt ist aus Kupfersulfat, Kupfercarbonat, Kupfer(II)-Lysin-Komplex, Kupfer(II)-Citrat und Kupfer(II)-Gluconat, oder wobei der Kupfersalz-Komplex ein Kupfer(I)-Salz-Komplex ist, der ausgewählt ist aus Kupfer(I)-Chlorid und Kupfer(I)-Acetat.

8. Peptid- oder Proteinarzneistoff zur Verwendung nach Anspruch 1 oder Zinksalz-Komplex zur Verwendung nach Anspruch 3 oder Reduktionsmittel zur Verwendung nach Anspruch 4 oder Arzneimittel zur Verwendung nach Anspruch 5 oder pharmazeutische Darreichungsform zur Verwendung nach Anspruch 6, wobei der Zinksalz-Komplex ein Zink(II)-Salz-Komplex ist, der ausgewählt ist aus Zinksulfat, Zinkchlorid, Zinkacetat, Zinkoxid, Zinkascorbat, Zinkcaprylat, Zinkgluconat, Zinkstearat und Zinkcarbonat.

9. Peptid- oder Proteinarzneistoff zur Verwendung nach einem der Ansprüche 1 , 7 oder 8 oder Kupfersalz-Komplex zur Verwendung nach Anspruch 2 oder 7 oder Zinksalz-Komplex zur Verwendung nach Anspruch 3 oder 8 oder Reduktionsmittel zur Verwendung nach einem der Ansprüche 4, 7 oder 8 oder Arzneimittel zur Verwendung nach einem der Ansprüche 5, 7 oder 8 oder pharmazeutische Darreichungsform zur Verwendung nach einem der Ansprüche 6 bis 8, wobei das Reduktionsmittel ausgewählt ist aus Ascorbinsäure, reduziertem Glutathion, Cystein, Harnsäure, einem reduzierenden Zucker, Glucose, Glyceraldehyd, Galactose, Lactose, Maltose, Mannit, α-Tocopherol, Vitamin A, α-Liponsäure, Dihydro-α-Liponsäure, einer ein Thiol tragenden Verbindung, einem Thiomer und pharmazeutisch verträglichen Salzen davon.

10. Peptid- oder Proteinarzneistoff zur Verwendung nach einem der Ansprüche 1 oder 7 bis 9 oder Kupfersalz-Komplex zur Verwendung nach einem der Ansprüche 2, 7 oder 9 oder Zinksalz-Komplex zur Verwendung nach einem der Ansprüche 3, 8 oder 9 oder Reduktionsmittel zur Verwendung nach einem der Ansprüche 4 oder 7 bis 9 oder Arzneimittel zur Verwendung nach einem der Ansprüche 5 oder 7 bis 9 oder pharmazeutische Darreichungsform zur Verwendung nach einem der Ansprüche 6 bis 9, wobei der Peptid- oder Proteinarzneistoff oder der Kupfersalz-Komplex oder der Zinksalz-Komplex oder das Reduktionsmittel oral in Kombination mit einem Absorptionsverstärker zu verabreichen ist, oder wobei das Arzneimittel oder die pharmazeutische Darreichungsform des Weiteren einen Absorptionsverstärker umfasst.

11. Peptid- oder Proteinarzneistoff zur Verwendung nach Anspruch 10 oder Kupfersalz-Komplex zur Verwendung nach Anspruch 10 oder Zinksalz-Komplex zur Verwendung nach Anspruch 10 oder Reduktionsmittel zur Verwendung nach Anspruch 10 oder Arzneimittel zur Verwendung nach Anspruch 10 oder pharmazeutische Darreichungsform zur Verwendung nach Anspruch 10, wobei der Absorptionsverstärker ausgewählt ist aus C₈₋₂₀-Alkanoyl-Carnitin, Salicylsäure, einem Salicylsäure-Derivat, 3-Methoxysalicylsäure, 5-Methoxysalicylsäure, Homovanillinsäure, einer C₈₋₂₀-Alkansäure, Citronensäure, einer mit einer Fettsäure acylierten Aminosäure, einem C₈₋₂₀-Alkanoyl-Sarcosinat, einem Alkylsaccharid, einem C₈₋₁₀-Alkylpolysaccharid, n-Octyl-beta-D-glucopyranosid, n-Dodecyl-beta-D-maltosid, einem Cyclodextrin, α-Cyclodextrin, β-Cyclodextrin, γ-Cyclodextrin, Methyl-β-cyclodextrin, Hydroxypropyl-β-cyclodextrin, Sulfobutylether-β-cyclodextrin, Natrium-N-[8-(2-hydroxybenzoyl)amino]caprylat, einem Thiomer, einer Calcium chelierenden Verbindung, Ethylendiamintetraessigsäure, Ethylenglycoltetraessigsäure, Polyacrylsäure, Cremophor EL, Chitosan, N,N,N-Trimethyl-Chitosan, Benzalkoniumchlorid, Bestatin, Cetylpyridiniumchlorid, Cetyltrimethylammoniumbromid, einem C₂₋₂₀-Alkanol, einem C₈₋₂₀-Alkenol, C₈₋₂₀-Alkensäure, Dextransulfat, Diethylenglycolmonoethylether, 1-Dodecyl-azacycloheptan-2-on, Ethylcaprylat, Glycerylmonolaurat, Lysophosphatidylcholin, Menthol, einem C₈₋₂₀-Alkylamin, einem C₈₋₂₀-Alkenylamin, Phosphatidylcholin, einem Poloxamer, Polyethylenglycolmonolaurat, Polyoxyethylen, Polypropylenglycolmonolaurat, einem Polysorbat, einem Deoxycholat, Natriumglycocholat, Natriumglycodeoxycholat, Natriumlaurylsulfat, einem Taurocholat, einem Taurodeoxycholat, Saccharoselaurat, einem Sulfoxid, Decylmethylsulfoxid, Dimethylsulfoxid, Cyclopentadecalacton, 8-(N-2-Hydroxy-5-chloro-benzoyl)aminocaprylsäure, Dodecyl-2-N,N-dimethyl-aminopropionat, D-α-Tocopherylpolyethylenglycol-1000-Succinat und pharmazeutisch verträglichen Salzen davon.

12. Peptid- oder Proteinarzneistoff zur Verwendung nach Anspruch 11 oder Kupfersalz-Komplex zur Verwendung nach Anspruch 11 oder Zinksalz-Komplex zur Verwendung nach Anspruch 11 oder Reduktionsmittel zur Verwendung nach Anspruch 11 oder Arzneimittel zur Verwendung nach Anspruch 11 oder pharmazeutische Darreichungsform zur Verwendung nach Anspruch 11, wobei der Absorptionsverstärker eine fettsäureacylierte Aminosäure ist ausgewählt aus Natriumlauroylalaninat, N-Dodecanoyl-L-Alanin, Natriumlauroylasparaginat, N-Dodecanoyl-L-Asparagin, Natriumlauroylaspariginsäure, N-Dodecanoyl-L-Aspariginsäure, Natrium lauroyl-cysteinat, N-Dodecanoyl-L-Cystein, Natriumlauroylglutaminsäure, N-Dodecanoyl-L-glutaminsäure, Natrium-lauroylglutaminat, N-Dode-canoyl-L-Glutamin, Natriumlauroylglycinat, N-Dodecanoyl-L-Glycin, Natrium-lauroylhistidinat, N-Dodecanoyl-L-Histidin, Natriumlauroylisoleucinat, N-Do-decanoyl-L-Isoleucin, Natriumlauroylleucinat, N-Dodecanoyl-L-Leucin, Natriumlauroylmethioninat, N-Dodecanoyl-L-Methionin, Natrium lauroylphenyl-alaninat, N-Dodecanoyl-L-Phenylalanin, Natriumlauroylprolinat, N-Dodecanoyl-L-Prolin, Natriumlauroylserinat, N-Dodecanoyl-L-Serin, Natrium lauroyl-threoninat, N-Dodecanoyl-L-Threonin, Natrium lauroyltryptophanat, N-Do-decanoyl-L-Tryptophan, Natriumlauroyltyrosinat, N-Dodecanoyl-L-Tyrosin, Natriumlauroylvalinat, N-Dodecanoyl-L-Valin, Natrium lauroylsarcosinat, N-Do-decanoyl-L-Sarcosin, Natriumcaprinalaninat, N-Decanoyl-L-Alanin, Natriumcaprinasparaginat, N-Decanoyl-L-Asparagin, Natriumcaprinaspartin-säure, N-Decanoyl-L-Aspartinsäure, Natriumcaprincysteinat, N-Decanoyl-L-Cystein, Natriumcapringlutaminsäure, N-Decanoyl-L-Glutaminsäure, Natriumcapringlutaminat, N-Decanoyl-L-Glutamin, Natriumcapringlycinat, N-Decanoyl-L-Glycin, Natriumcaprinhistidinat, N-Decanoyl-L-Histidin, Natriumcaprinisoleucinat, N-Decanoyl-L-Isoleucin, Natriumcaprinleucinat, N-Decanoyl-L-Leucin, Natriumcaprinmethioninat, N-Decanoyl-L-Methionin, Natriumcaprin-phenylalaninat, N-Decanoyl-L-Phenylalanin, Natriumcaprinprolinat, N-Decanoyl-L-Prolin, Natriumcaprinserinat, N-Decanoyl-L-Serin, Natriumcaprinthreoninat, N-Decanoyl-L-Threonin, Natriumcaprintryptophanat, N-Decanoyl-L-Tryptophan, Natriumcaprintyrosinat, N-Decanoyl-L-Tyrosin, Natriumcaprinvalinat, N-Decanoyl-L-Valin, Natriumcaprinsarcosinat, N-Decanoyl-L-Sarcosin, Natriumoleoylsarcosinat, Natrium-N-Decylleucin, Natriumstearoylglutamat, Natriummyristoylglutamat, Natrium lauroylglutamat, Natriumcocoylglutamat, Natriumcocoylglycinat, Natrium-N-Decylleucin, Natriumcocoylglycin, Natriumcocoylglutamat, Natrium lauroylalaninat, N-Do-decanoyl-L-Alanin, Natrium lauroylasparaginat, N-Dodecanoyl-L-Asparagin, Natrium lauroylasparaginsäure, N-Dodecanoyl-L-Asparaginsäure, Natriumlauroylcysteinat, N-Dodecanoyl-L-Cystein, Natriumlauroylglutaminsäure, N-Do-decanoyl-L-Glutaminsäure, Natriumlauroylglutaminat, N-Dodecanoyl-L-Glutamin, Natriumlauroylglycinat, N-Dodecanoyl-L-Glycin, Natrium-lauroylhistidinat, N-Dodecanoyl-L-Histidin, Natrium lauroylisoleucinat, N-Do-decanoyl-L-Isoleucin, Natrium lauroylleucinat, N-Dodecanoyl-L-Leucin, Natrium lauroylmethioninat, N-Dodecanoyl-L-Methionin, Natriumlauroylphenylalaninat, N-Dodecanoyl-L-Phenylalanin, Natriumlauroylprolinat, N-Dodecanoyl-L-Prolin, Natriumlauroylserinat, N-Dodecanoyl-L-Serin, Natriumlauroylthreoninat, N-Dodecanoyl-L-Threonin, Natrium lauroyltryptophanat, N-Do-decanoyl-L-Tryptophan, Natrium lauroyltyrosinat, N-Dodecanoyl-L-Tyrosin, Natrium lauroylvalinat, N-Dodecanoyl-L-Valin, N-Dodecanoyl-L-Sarcosin, Natriumcaprinalaninat, N-Decanoyl-L-Alanin, Natriumcaprinasparaginat, N-Decanoyl-L-Asparagin, Natriumcaprinasparaginsäure, N-Decanoyl-L-Asparaginsäure, Natriumcaprincysteinat, N-Decanoyl-L-Cystein, Natriumcapringlutaminsäure, N-Decanoyl-L-Glutaminsäure, Natriumcapringlutaminat, N-Decanoyl-L-Glutamin, Natriumcapringlycinat, N-Decanoyl-L-Glycin, Natriumcaprinhistidinat, N-Decanoyl-L-Histidin, Natriumcaprinisoleucinat, N-Decanoyl-L-Isoleucin, Natriumcaprinleucinat, N-Decanoyl-L-Leucin, Natriumcaprinmethioninat, N-Decanoyl-L-Methionin, Natriumcaprin-phenylalaninat, N-Decanoyl-L-Phenylalanin, Natriumcaprinprolinat, N-Decanoyl-L-Prolin, Natriumcaprinserinat, N-Decanoyl-L-Serin, Natriumcaprinthreoninat, N-Decanoyl-L-Threonin, Natriumcaprintryptophanat, N-Decanoyl-L-Tryptophan, Natriumcaprintyrosinat, N-Decanoyl-L-Tyrosin, Natriumcaprinvalinat, N-Decanoyl-L-Valin, Natriumcaprinsarcosinat, Natriumoleoylsarcosinat und pharmazeutisch verträglichen Salzen davon.

13. Arzneimittel zur Verwendung nach einem der Ansprüche 10 bis 12 oder pharmazeutische Darreichungsform zur Verwendung nach einem der Ansprüche 10 bis 12, wobei das Arzneimittel oder die pharmazeutische Darreichungsform umfasst:
den Kupfersalz-Komplex in einer Menge von etwa 0,1 mg bis etwa 5 mg pro Dosierungseinheit;
und/oder den Zinksalz-Komplex in einer Menge von etwa 0,1 mg bis etwa 50 mg pro Dosierungseinheit;
das Reduktionsmittel in einer Menge von etwa 1 mg bis etwa 1000 mg pro Dosierungseinheit; und
den Absorptionsverstärker in einer Menge von etwa 10 mg bis etwa 1000 mg pro Dosierungseinheit.

## Revendications

1. Substance pharmaceutique peptidique ou protéique pour utilisation en tant que médicament, laquelle substance pharmaceutique peptidique ou protéique est destinée à être administrée par voie orale en combinaison avec :
un sel/complexe de cuivre pharmaceutiquement acceptable et/ou un sel/ complexe de zinc pharmaceutiquement acceptable ; et
un agent réducteur pharmaceutiquement acceptable ;
où la substance pharmaceutique peptidique ou protéique :
(i) est une substance pharmaceutique peptidique ou protéique ayant un poids moléculaire d'environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine, le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

2. Sel/complexe de cuivre pharmaceutiquement acceptable pour utilisation en thérapie, lequel sel/complexe de cuivre est destiné à être administré par voie orale en combinaison avec :
un agent réducteur pharmaceutiquement acceptable ; et
une substance pharmaceutique peptidique ou protéique qui :
(i) est une substance pharmaceutique peptidique ou protéique ayant un poids moléculaire d'environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine, le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

3. Sel/complexe de zinc pharmaceutiquement acceptable pour utilisation en thérapie, lequel sel/complexe de zinc est destiné à être administré par voie orale en combinaison avec :
un agent réducteur pharmaceutiquement acceptable ; et
une substance pharmaceutique peptidique ou protéique qui :
(i) est une substance pharmaceutique peptidique ou protéique ayant un poids moléculaire d'environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine, le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

4. Agent réducteur pharmaceutiquement acceptable pour utilisation en thérapie, lequel agent réducteur est destiné à être administré par voie orale en combinaison avec :
un sel/complexe de cuivre pharmaceutiquement acceptable et/ou un sel/ complexe de zinc pharmaceutiquement acceptable ; et
une substance pharmaceutique peptidique ou protéique qui :
(i) est une substance pharmaceutique peptidique ou protéique ayant un poids moléculaire d'environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine, le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

5. Composition pharmaceutique pour utilisation en thérapie par administration orale comprenant :
une substance pharmaceutique peptidique ou protéique;
un sel/complexe de cuivre pharmaceutiquement acceptable et/ou un sel/ complexe de zinc pharmaceutiquement acceptable ; et
un agent réducteur pharmaceutiquement acceptable ; où la substance pharmaceutique peptidique ou protéique :
(i) est une substance pharmaceutique peptidique ou protéique ayant un poids moléculaire d'environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine,
le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

6. Forme posologique pharmaceutique pour utilisation en thérapie par administration orale comprenant :
une substance pharmaceutique peptidique ou protéique;
un sel/complexe de cuivre pharmaceutiquement acceptable et/ou un sel/ complexe de zinc pharmaceutiquement acceptable ; et
un agent réducteur pharmaceutiquement acceptable ;
dans laquelle la substance pharmaceutique peptidique ou protéique est physiquement séparée du sel/complexe de cuivre pharmaceutiquement acceptable et du sel/complexe de zinc pharmaceutiquement acceptable à l'intérieur de la forme posologique pharmaceutique ;
dans lequel la substance pharmaceutique peptidique ou protéique :
(i) est une substance pharmaceutique peptidique ou protéique ayant un poids moléculaire inférieur ou égal à environ 2 kDa à environ 5 kDa, qui n'est pas le glucagon ; ou
(ii) est une substance pharmaceutique peptidique ou protéique ayant un poids moléculaire inférieur ou égal à environ 50 kDa, qui est sélectionnée parmi GLP-1, un analogue de GLP-1, un analogue de GLP-1 acylé, un analogue de GLP-1 diacylé, le sémaglutide, le liraglutide, l'exénatide, le lixizénatide, un agoniste double du récepteur de GLP-1 et du récepteur de glucagon, l'amyline, un analogue d'amyline, le pramlintide, un analogue de somatostatine, l'octréotide, le lanréotide, le pasiréotide, la goséréline, la buséréline, la leptine, un analogue de leptine, la métréleptine, le peptide YY, un analogue de peptide YY, le glatiramère, le leuprolide, le tériparatide, la desmopressine, un antibiotique glycopeptidique, un antibiotique peptidique non ribosomial cyclique ou polycyclique glycosylé, la vancomycine, la téicoplanine, la télavancine, la bléomycine, la ramoplanine, la décaplanine, le bortézomib, la cosyntropine, la gonadotropine chorionique, la ménotropine, la sermoréline, la gonadolibérine, la calcitonine, la calcitonine de saumon, la pentagastrine, l'oxytocine, le néséritide, l'anakinra, l'enfuvirtide, le pegvisomant, la dornase alfa, la lépirudine, l'anidulafungine, l'eptifibatide, l'interféron alfacon-1, l'interféron bêta-1a, l'interféron bêta-1b, le PEG-interféron alfa-2a, le PEG-interféron alfa-2b, le PEG-interféron bêta-1a, la fibrinolysine, la vasopressine, l'aldesleukine, la darbépoétine alfa, le filgrastim, l'interleukine-11, la cyclosporine, l'urokinase, la viomycine, la thyréolibérine, le leucine-enképhaline, la méthionine-enképhaline, la substance P, la corticostimuline, la parathormone, et leurs sels pharmaceutiquement acceptables.

7. Substance pharmaceutique peptidique ou protéique pour utilisation selon revendication 1, ou sel/complexe de cuivre pour utilisation selon revendication 2, ou agent réducteur pour utilisation selon revendication 4, ou composition pharmaceutique pour utilisation selon revendication 5, ou forme posologique pharmaceutique pour utilisation selon revendication 6, dans lequel ledit sel/complexe de cuivre est un sel/complexe de cuivre(II) choisi parmi le sulfate de cuivre, le carbonate de cuivre, un complexe de lysine et de cuivre(II), le citrate de cuivre(II), et le gluconate de cuivre(II), ou bien ledit sel/complexe de cuivre est un sel/complexe de cuivre(I) choisi parmi le chlorure de cuivre(I) et l'acétate de cuivre(I).

8. Substance pharmaceutique peptidique ou protéique pour utilisation des revendication 1, ou sel/complexe de zinc pour utilisation selon revendication 3, ou agent réducteur pour utilisation selon revendication 4, ou composition pharmaceutique pour utilisation selon revendication 5, ou forme posologique pharmaceutique pour utilisation selon revendication 6, dans lequel ledit sel/complexe de zinc est un sel/complexe de zinc(II) choisi parmi le sulfate de zinc, le chlorure de zinc, l'acétate de zinc, l'oxyde de zinc, l'ascorbate de zinc, le caprylate de zinc, le gluconate de zinc, le stéarate de zinc, et le carbonate de zinc.

9. Substance pharmaceutique peptidique ou protéique pour utilisation selon l'une quelconque des revendications 1, 7 ou 8, ou sel/complexe de cuivre pour utilisation selon revendication 2 ou 7, ou sel/complexe de zinc pour utilisation selon revendication 3 ou 8, ou agent réducteur pour utilisation selon l'une quelconque des revendications 4, 7 ou 8, ou composition pharmaceutique pour utilisation selon l'une quelconque des revendications 5, 7 ou 8, ou forme posologique pharmaceutique pour utilisation selon l'une quelconque des revendications 6 à 8, dans lequel ledit agent réducteur est choisi parmi l'acide ascorbique, le glutathion réduit, la cystéine, l'acide urique, un sucre réducteur, le glucose, le glycéraldéhyde, le galactose, le lactose, le maltose, le mannitol, l'a-tocophérol, la vitamine A, l'acide α-lipoïque, l'acide dihydro-α-lipoïque, un composé portant un thiol, un thiomère, et leurs sels pharmaceutiquement acceptables.

10. Substance pharmaceutique peptidique ou protéique pour utilisation selon l'une quelconque des revendications 1 ou 7 à 9, ou sel/complexe de cuivre pour utilisation selon l'une quelconque des revendications 2, 7 ou 9, ou sel/complexe de zinc pour utilisation selon l'une quelconque des revendications 3, 8, ou 9, ou agent réducteur pour utilisation selon l'une quelconque des revendications 4 ou 7 à 9, ou composition pharmaceutique pour utilisation selon l'une quelconque des revendications 5 ou 7 à 9, ou forme posologique pharmaceutique pour utilisation selon l'une quelconque des revendications 6 à 9, dans lequel ladite substance pharmaceutique peptidique ou protéique ou ledit sel/ complexe de cuivre ou ledit sel/complexe de zinc ou ledit agent réducteur est destiné à être administré par voie orale en combinaison avec un amplificateur d'absorption, ou dans lequel ladite composition pharmaceutique ou ladite forme posologique pharmaceutique comprend en outre un amplificateur d'absorption.

11. Substance pharmaceutique peptidique ou protéique pour utilisation selon la revendication 10 ou sel/complexe de cuivre pour utilisation selon la revendication 10 ou sel/complexe de zinc pour utilisation selon la revendication 10 ou agent réducteur pour utilisation selon la revendication 10 ou composition pharmaceutique pour utilisation selon la revendication 10 ou forme posologique pharmaceutique pour utilisation selon la revendication 10, dans lequel ledit amplificateur d'absorption est choisi parmi une (alcanoyle en C₈ à C₂₀)carnitine, l'acide salicylique, un dérivé d'acide salicylique, l'acide 3-méthoxysalicylique, l'acide 5-méthoxysalicylique, l'acide homovanillique, un acide alcanoïque en C₈ à C₂₀, l'acide citrique, un acide aminé acylé avec un acide gras, un sarcosinate d'alcanoyle en C₈ à C₂₀), un alkylsaccharide, un (alkyle en C₈ à C₁₀)polysaccharide, le n-octyl-β-D-glucopyranoside, le n-dodécyl-β-D-maltoside, une cyclodextrine, l'a-cyclodextrine, la β-cyclodextrine, la γ-cydodextrine, la méthyl-β-cyclodextrine, l'hydroxypropyl-β-cyclodextrine, la sulfobutyléther-β-cyclodextrine, le N-[8-(2-hydroxybenzoyl)amino]caprylate de sodium, un thiomère, un composé chélatant le calcium, l'acide éthylènediaminetétraacétique, l'acide éthylèneglycol-tétraacétique, le poly(acide acrylique), le crémophor EL, le chitosan, le N,N,N-triméthylchitosan, le chlorure de benzalkonium, la bestatine, le chlorure de cétylpyridinium, le bromure de cétyltriméthylammonium, un alcanol en C₂ à C₂₀, un alcénol en C₈ à C₂₀, un acide alcénoïque en C₈ à C₂₀, le sulfate de dextrane, l'éther monoéthylique de diéthylèneglycol, la 1-dodécylazacycloheptan-2-one, le caprylate d'éthyle, le monolaurate de glycéryle, la lysophosphatidylcholine, le menthol, une alkylamine en C₈ à C₂₀, une alcénylamine en C₈ à C₂₀, la phosphatidylcholine, un poloxamère, le monolaurate de polyéthylèneglycol, polyoxyéthyléné, le monolaurate de polypropylèneglycol, un polysorbate, un désoxycholate, le glycocholate de sodium, le glycodésoxycholate de sodium, le laurylsulfate de sodium, un taurocholate, un taurodésoxycholate, le laurate de saccharose, un sulfoxyde, le décylméthylsulfoxyde, le diméthylsulfoxyde, la cyclopentadécalactone, l'acide 8-(N-2-hydroxy-5-chlorobenzoyl)aminocaprylique, le 2-N,N-diméthylaminopropionate de dodécyle, l'adduit de succinate de D-α-tocophéryle et de polyéthylèneglycol-1000, et leurs sels pharmaceutiquement acceptables.

12. Substance pharmaceutique peptidique ou protéique pour utilisation selon la revendication 11 ou sel/complexe de cuivre pour utilisation selon la revendication 11 ou sel/complexe de zinc pour utilisation selon la revendication 11 ou agent réducteur pour utilisation selon la revendication 11 ou composition pharmaceutique pour utilisation selon la revendication 11 ou forme posologique pharmaceutique pour utilisation selon la revendication 11, dans lequel ledit amplificateur d'absorption est un acide aminé acylé avec un acide gras choisi parmi le lauroyl-alaninate de sodium, la N-dodécanoyl-L-alanine, le lauroyl-asparaginate de sodium, la N-dodécanoyl-L-asparagine, l'acide sodium-lauroyl-aspartique, l'acide N-dodécanoyl-L-aspartique, le lauroyl-cystéinate de sodium, la N-dodécanoyl-L-cystéine, l'acide sodium-lauroyl-glutamique, l'acide N-dodécanoyl-L-glutamique, le lauroyl-glutaminate de sodium, la N-dodécanoyl-L-glutamine, le lauroyl-glycinate de sodium, la N-dodécanoyl-L-glycine, le lauroyl-histidinate de sodium, la N-dodécanoyl-L-histidine, le lauroyl-isoleucinate de sodium, la N-dodécanoyl-L-isoleucine, le lauroyl-leucinate de sodium, la N-dodécanoyl-L-leucine, le lauroyl-méthioninate de sodium, la N-dodécanoyl-L-méthionine, le lauroyl-phénylalaninate de sodium, la N-dodécanoyl-L-phénylalanine, le lauroyl-prolinate de sodium, la N-dodécanoyl-L-proline, le lauroyl-sérinate de sodium, la N-dodécanoyl-L-sérine, le lauroyl-thréoninate de sodium, la N-dodécanoyl-L-thréonine, le lauroyl-tryptophanate de sodium, le N-dodécanoyl-L-tryptophane, le lauroyl-tyrosinate de sodium, la N-dodécanoyl-L-tyrosine, le lauroyl-valinate de sodium, la N-dodécanoyl-L-valine, le lauroyl-sarcosinate de sodium, la N-dodécanoyl-L-sarcosine, l'alaninate de sodium caprique, la N-décanoyl-L-alanine, l'asparaginate de sodium caprique, la N-décanoyl-L-asparagine, l'acide caprique-aspartique sodique, l'acide N-décanoyl-L-aspartique, le cystéinate de sodium caprique, la N-décanoyl-L-cystéine, l'acide captique-glutamique sodique, l'acide N-décanoyl-L-glutamique, le glutaminate de sodium caprique, la N-décanoyl-L-glutamine, le glycinate de sodium caprique, la N-décanoyl-L-glycine, l'histidinate de sodium caprique, la N-décanoyl-L-histidine, l'isoleucinate de sodium caprique, la N-décanoyl-L-isoleucine, le leucinate de sodium caprique, la N-décanoyl-L-leucine, le méthioninate de sodium caprique, la N-décanoyl-L-méthionine, le phénylalaninate de sodium caprique, la N-décanoyl-L-phénylalanine, le prolinate de sodium caprique, la N-décanoyl-L-proline, le sérinate de sodium caprique, la N-décanoyl-L-sérine, le thréoninate de sodium caprique, la N-décanoyl-L-thréonine, le tryptophanate de sodium caprique, le N-décanoyl-L-tryptophane, le tyrosinate de sodium caprique, la N-décanoyl-L-tyrosine, le valinate de sodium caprique, la N-décanoyl-L-valine, le sarcosinate de sodium caprique, la N-décanoyl-L-sarcosine, l'oléoyl-sarcosinate de sodium, la N-décyl-leucine sodique, le stéaroyl-glutamate de sodium, le myristoyl-glutamate de sodium, le lauroyl-glutamate de sodium, le (coprah-yl)glutamate de sodium, le (coprah-yl)glycinate de sodium, la N-décyl-leucine sodique, la (coprah-yl)glycine sodique, le (coprah-yl)glutamate de sodium, le lauroyl-alaninate de sodium, la N-dodécanoyl-L-alanine, le lauroyl-asparaginate de sodium, la N-dodécanoyl-L-asparagine, l'acide sodium-lauroyl-aspartique, l'acide N-dodécanoyl-L-aspartique, le lauroyl-cystéinate de sodium, la N-dodécanoyl-L-cystéine, l'acide sodium-lauroyl-glutamique, l'acide N-dodécanoyl-L-glutamique, le lauroyl-glutaminate de sodium, la N-dodécanoyl-L-glutamine, le lauroyl-glycinate de sodium, la N-dodécanoyl-L-glycine, le lauroyl-histidinate de sodium, la N-dodécanoyl-L-histidine, le lauroyl-isoleucinate de sodium, la N-dodécanoyl-L-isoleucine, le lauroyl-leucinate de sodium, la N-dodécanoyl-L-leucine, le lauroyl-méthinoninate de sodium, la N-dodécanoyl-L-méthionine, le lauroyl-phénylalaninate de sodium, la N-dodécanoyl-L-phénylalanine, le lauroyl-prolinate de sodium, la N-dodécanoyl-L-proline, le lauroyl-sérinate de sodium, la N-dodécanoyl-L-sérine, le lauroyl-thréoninate de sodium, la N-dodécanoyl-L-thréonine, le lauroyl-tryptophanate de sodium, le N-dodécanoyl-L-tryptophane, le lauroyl-tyrosinate de sodium, la N-dodécanoyl-L-tyrosine, le lauroyl-valinate de sodium, la N-dodécanoyl-L-valine, la N-dodécanoyl-L-sarcosine, l'alaninate de sodium caprique, la N-décanoyl-L-alanine, l'asparaginate de sodium caprique, la N-décanoyl-L-asparagine, l'acide caprique-aspartique sodique, l'acide N-décanoyl-L-aspartique, le cystéinate de sodium caprique, la N-décanoyl-L-cystéine, l'acide caprique-glutamique sodique, l'acide N-décanoyl-L-glutamique, le glutaminate de sodium caprique, la N-décanoyl-L-glutamine, le glycinate de sodium caprique, la N-décanoyl-L-glycine, l'histidinate de sodium caprique, la N-décanoyl-L-histidine, l'isoleucinate de sodium caprique, la N-décanoyl-L-isoleucine, le leucinate de sodium caprique, la N-décanoyl-L-leucine, le méthioninate de sodium caprique, la N-décanoyl-L-méthionine, le phénylalaninate de sodium caprique, la N-décanoyl-L-phénylalanine, le prolinate de sodium caprique, la N-décanoyl-L-proline, le sérinate de sodium caprique, la N-décanoyl-L-sérine, le thréoninate de sodium caprique, la N-décanoyl-L-thréonine, le tryptophanate de sodium caprique, le N-décanoyl-L-tryptophane, le tyrosinate de sodium caprique, la N-décanoyl-L-tyrosine, le valinate de sodium caprique, la N-décanoyl-L-valine, le sarcosinate de sodium caprique, l'oléoyl-sarcosinate de sodium, et leurs sels pharmaceutiquement acceptables.

13. Composition pharmaceutique pour utilisation selon l'une quelconque des revendications 10 à 12 ou forme posologique pharmaceutique pour utilisation selon l'une quelconque des revendications 10 à 12, laquelle composition pharmaceutique ou forme posologique pharmaceutique comprend :
le sel/complexe de cuivre en une quantité d'environ 0,1 mg à environ 5 mg par unité posologique ;
et/ou le sel/complexe de zinc en une quantité d'environ 0,1 mg à environ 50 mg par unité posologique ;
l'agent réducteur en une quantité d'environ 1 mg à environ 1000 mg par unité posologique ; et
l'amplificateur d'absorption en une quantité d'environ 10 mg à environ 1000 mg par unité posologique.
